# EUROPEAN PATENT APPLICATION

(11) **EP 2 103 935 A1**
(43) Date of publication of application: **23.09.2009**
(21) Application number: 09250599.9
(22) Date of filing: 02.03.2009
(51) Int. Cl.: G01N 33/50

(54) **Multiple administrations of umbilicus derived cells**

(30) Priority: 28.02.2008 US 39480
(71) Applicant: Centocor Ortho Biotech, Inc., Horsham, PA 19044 (US)
(72) Inventor: Sachs, David H., Aubumdale, MA 02466-2808 (US); Huang, Christene, Dover, MA 02030 (US); Cho, Patricia S., Boston, MA 02210 (US); Popma, Sicco H., Chalfont, PA 18914 (US)
(74) Representative: Marshall, Cameron John

(57) **Abstract**

A method to determine the optimal administration protocol of allogenic donor tissue to treat a disease in a human, using an animal model of human disease.

## Description

### FIELD OF THE INVENTION

The present invention provides methods to determine the optimal administration of umbilicus-derived cells into a recipient, using an animal model of human disease. The present invention also provides methods for multiple administrations of umbilicus-derived cells into a recipient.

### BACKGROUND

The adaptive immune system provides the recipient with the ability to recognize and remember specific pathogens (to generate immunity), and to mount stronger attacks each time the pathogen is encountered. Normally, the adaptive immune response protects against infection. However, the adaptive immune system can also have detrimental effects, such as, for example, recognizing transplanted tissue and eliciting an immune response against the donor tissue, leading to rejection. This detrimental response may be more pronounced after subsequent transplantation of donor tissue, as a result of the immune response mediated by the recipient's memory cells.

The cells of the adaptive immune system are special types of leukocytes, called lymphocytes. B cells and T cells are the major types of lymphocytes and are derived from pluripotent hemopoietic stem cells in the bone marrow. B cells are involved in the humoral immune response, whereas T cells are involved in the cellular immune response. Both B cells and T cells carry receptor molecules that recognize specific targets. T cells recognize a "non-self" target, such as a pathogen, only after antigens (small fragments of the pathogen) have been processed and presented in combination with a "self" receptor called major histocompatibility complex (MHC) molecules. There are two major subtypes of T cells: the killer T cell (CD8⁺) and the helper T cell (CD4⁺). Killer T cells only recognize antigens coupled to Class I MHC molecules, while helper T cells only recognize antigens coupled to Class II MHC molecules.

The transplantation of tissues and organs between two unrelated individuals may result in graft rejection, unless immunosuppressive therapy is given to control the immune response. The immunological barriers to successful stem cell transplantation are the same as those for tissue allografts obtained from conventional sources. Rejection occurs because of human leukocyte antigen (HLA) disparities in the major histocompatibility (MHC) molecules between donor and recipient. MHC class I molecules consist of HLA-A, HLA-B and HLA-C, and MHC class II molecules consist of HLA-DR, HLA-DQ and HLA-DP.

The lack of expression of MHC class II on the surface of donor tissue may suggest that the tissue is less immunogenic. However, the local *in vivo* environment at the transplant site can influence MHC class II expression. For example, inflammatory cytokines, such as interferon-γ (IFN-γ), are known to induce MHC class II expression (Le Blanc et al., Exp. Hematol. 31: 890- 96, 2003) and increase MHC I expression. Therefore, there is a significant need to understand the mechanisms by which donor MHC class II expression is regulated in the donor tissue and is influenced by the recipient's response after injection in order to develop protocols that minimize rejection of the donor tissue.

### SUMMARY

In one embodiment, the present invention provides methods to determine the optimal administration protocol of allogeneic donor tissue to treat a disease in a human, using an animal model of human disease, comprising the steps of:
a. Obtaining allogeneic donor tissue from a donor animal,
b. Inducing a disease state in a recipient animal,
c. Administering the allogeneic donor tissue to the recipient animal, and monitoring the immune response elicited by the allogeneic donor tissue, and
d. Altering the administration of the allogeneic donor tissue to reduce the immune response in the recipient animal.

In one embodiment, the present invention provides methods to determine the optimal administration protocol of allogeneic donor tissue to treat a disease in a human, using an animal model of human disease, comprising the steps of:
a. Obtaining allogeneic donor tissue from a donor animal,
b. Inducing a disease state in a recipient animal,
c. Administering the allogeneic donor tissue to the recipient animal, and monitoring the immune response elicited by the allogeneic donor tissue,
d. Monitoring the efficacy of the allogeneic donor tissue in the recipient animal, and
e. Altering the administration of the allogeneic donor tissue to reduce the immune response in the recipient animal.

In one embodiment, the disease state in the recipient animal is induced experimentally.

In one embodiment, the disease state in the recipient animal is genetic.

In one embodiment, the immune response elicited by the allogeneic donor tissue is any immune response.

In one embodiment, the immune response elicited by the allogeneic donor tissue is rejection of the allogeneic donor tissue.

In one embodiment, the immune response elicited by the allogeneic donor tissue is an inflammatory response. In one embodiment, the inflammatory response is mediated by interferon-γ. In one embodiment, the inflammatory response that is mediated by interferon-γ is an increase in the expression of MHC class II on the allogeneic donor tissue. In one embodiment, the inflammatory response that is mediated by interferon-γ is an increase in the expression of MHC class I and II on the allogeneic donor tissue.

In one embodiment, the administration is altered by altering the amount of allogeneic donor tissue administered to the recipient animal. In one embodiment, the amount of allogeneic donor tissue per administration is altered. In one embodiment, the number of administrations of allogenic donor tissue is altered.

In one embodiment, the administration is altered by altering the timing of the administration of the allogeneic donor tissue administered to the recipient animal. In one embodiment, timing of the initial administration to the recipient animal is altered. In one embodiment, the timing of the subsequent administrations is altered.

In one embodiment, the administration is altered by altering the site of the administration of the allogeneic donor tissue administered to the recipient animal.

In one embodiment, the recipient animal is treated with at least one agent that reduces the immune response elicited by the allogeneic donor tissue. In one embodiment, the recipient animal is treated systemically. In one embodiment, the recipient animal is treated at the site of administration. In one embodiment, the recipient animal is treated prior to administration of the allogeneic donor tissue. In one embodiment, the recipient animal is treated after the administration of the allogeneic donor tissue.

In one embodiment, the allogeneic donor tissue is treated with at least one agent that reduces the immune response elicited by the allogeneic donor tissue.

In one embodiment, the immune response observed in the recipient animal in response to the allogeneic donor tissue is predicted to be the same as the probable immune response observed in the recipient human in response to the allogeneic donor tissue.

In one embodiment, the allogeneic donor tissue obtained from a donor animal is equivalent to the allogeneic donor tissue obtained from a donor human.

In one embodiment; the optimal administration protocol for the recipient animal is used to administer allogeneic donor tissue to a human recipient.

In one embodiment, the allogeneic donor tissue comprises cells. In one embodiment, the cells are umbilicus-derived cells.

### BRIEF DESCRIPTION OF THE DRAWINGS

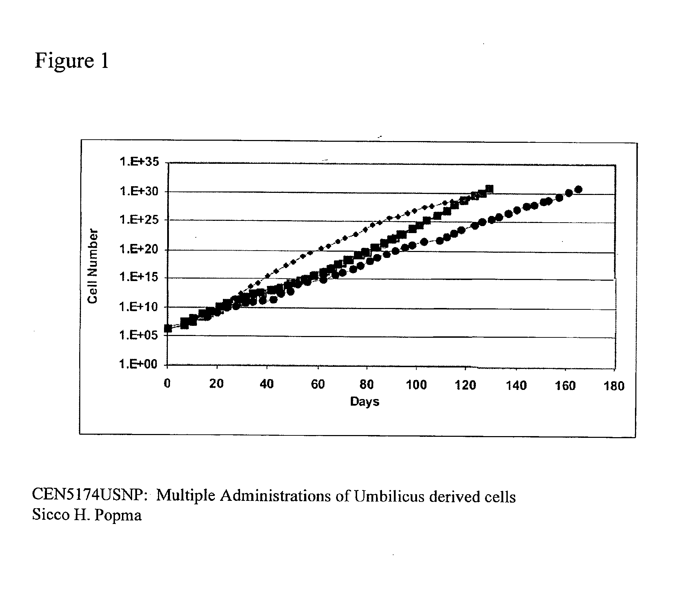

Figure 1 shows long-term growth curves of human umbilicus-derived cells and porcine umbilicus-derived cells from three different umbilical cords harvested from DD haplotype mini swine (donors 030105 DD1, DD3, and DD4).

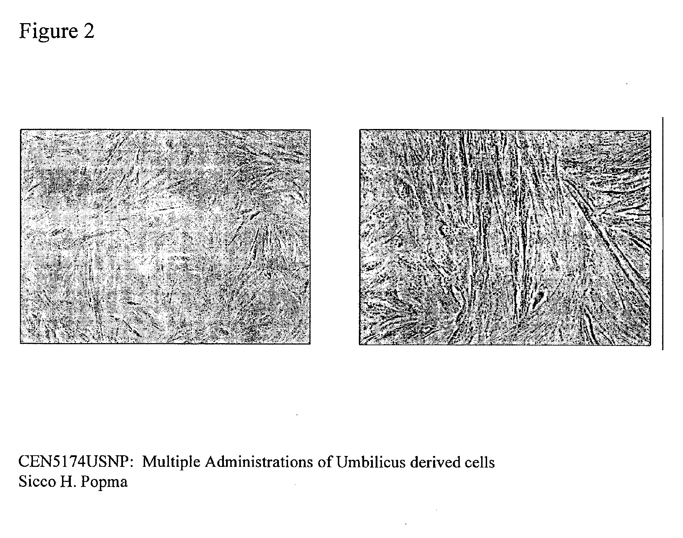

Figure 2 shows representative microphotograph images, 100x and 200x respectively, of porcine umbilicus-derived cells.

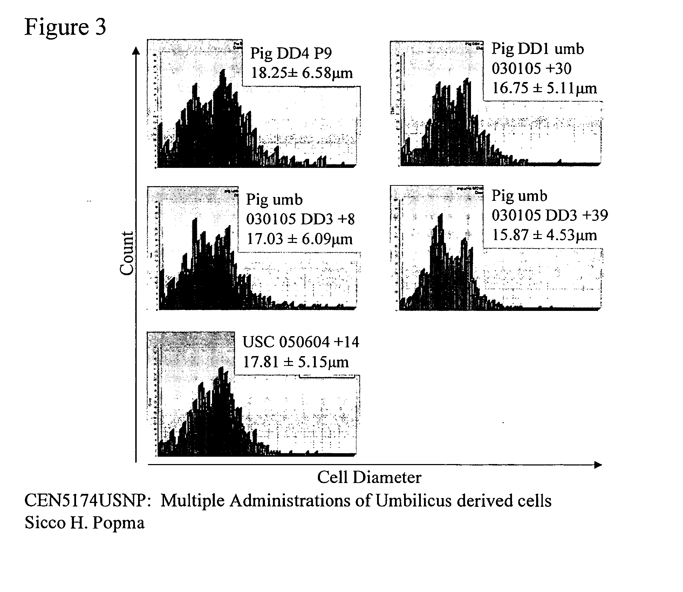

Figure 3 shows representative cell size histograms of cultured porcine umbilicus-derived cells from donors DD4 (P9), DD1 (P30), DD3 (P8, P39), and cultured human umbilicus-derived cells.

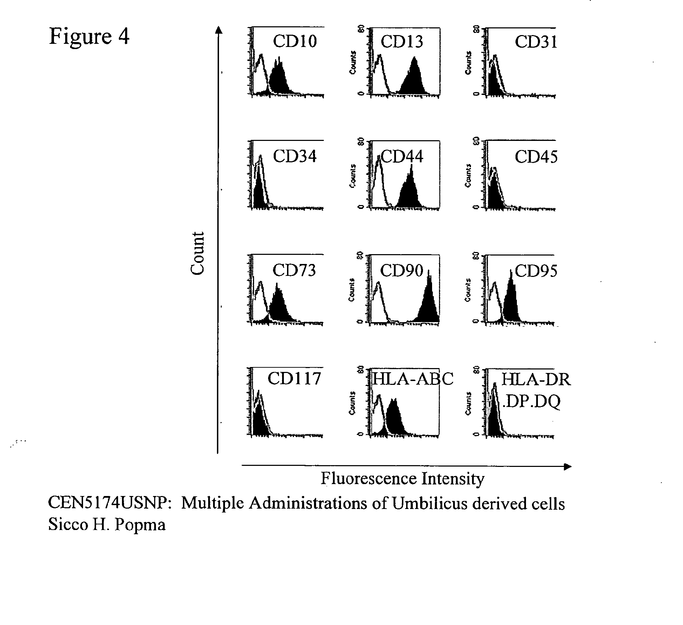

Figure 4 shows the expression of various cell surface markers on human umbilicus-derived cells. Flow cytometric results shown are representative histograms of cultured human umbilicus-derived cells (filled in traces). Isotype (negative) controls are shown by the in non filled in traces.

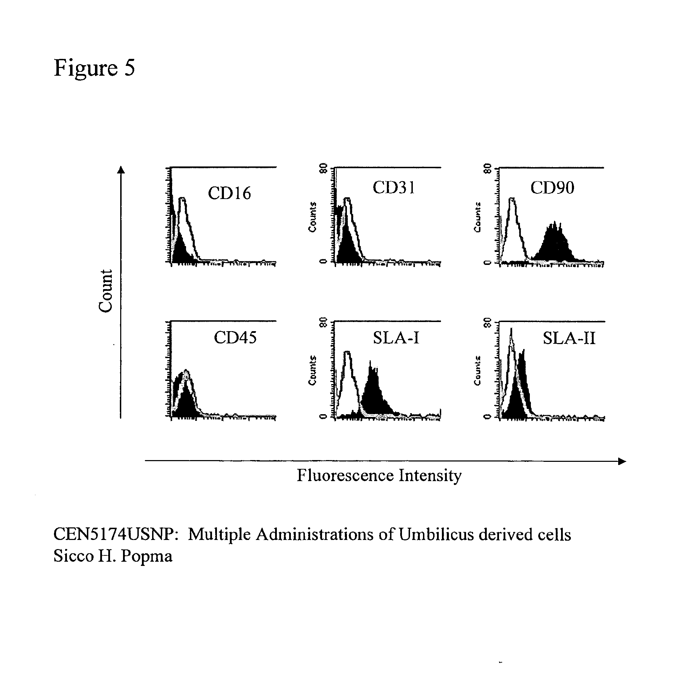

Figure 5 shows the expression of various cell surface markers on porcine umbilicus-derived cells. Flow cytometric results shown are representative histograms of cultured porcine umbilicus-derived cells (filled in tracer). Isotype (negative) controls are shown by the in non filled in traces.

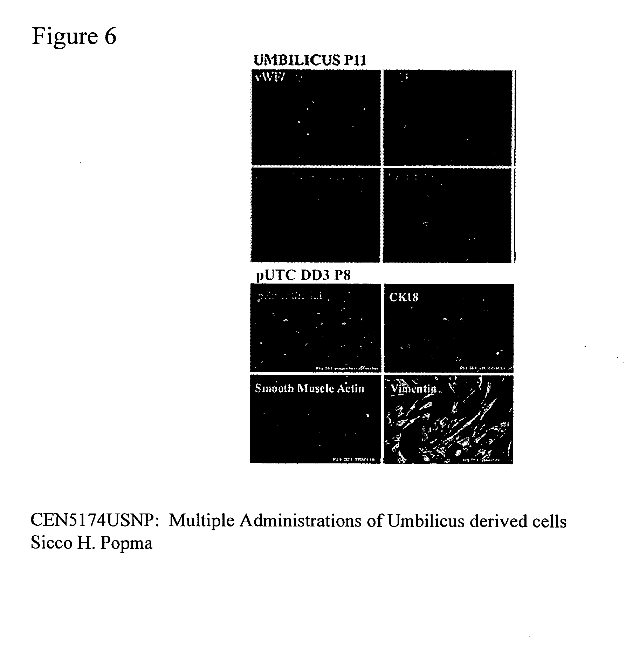

Figure 6 shows representative flow cytometric histograms of porcine umbilicus-derived cells analyzed with human (top) and rat (bottom) specific cell surface markers to exclude the presence of human or rat UTC in the pig UTC.

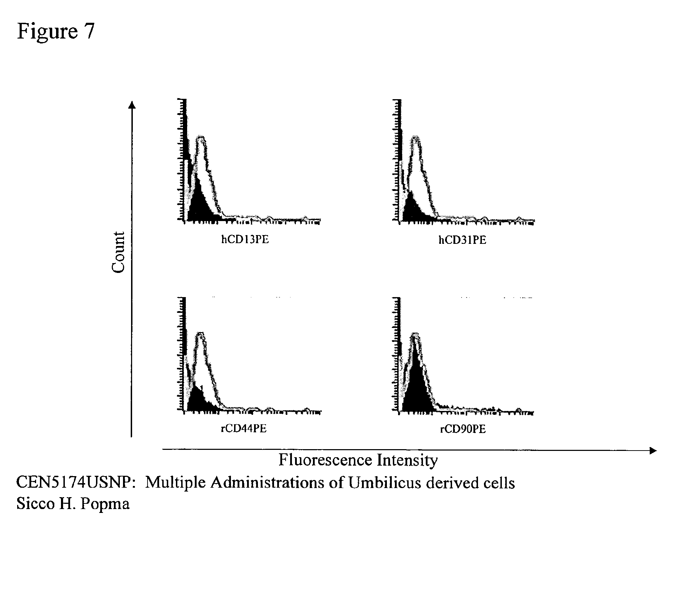

Figure 7 shows microphotographs of the expression of various proteins by immunocytochemistry in human umbilicus-derived cells (top panel) and porcine umbilicus-derived cells (bottom panel).

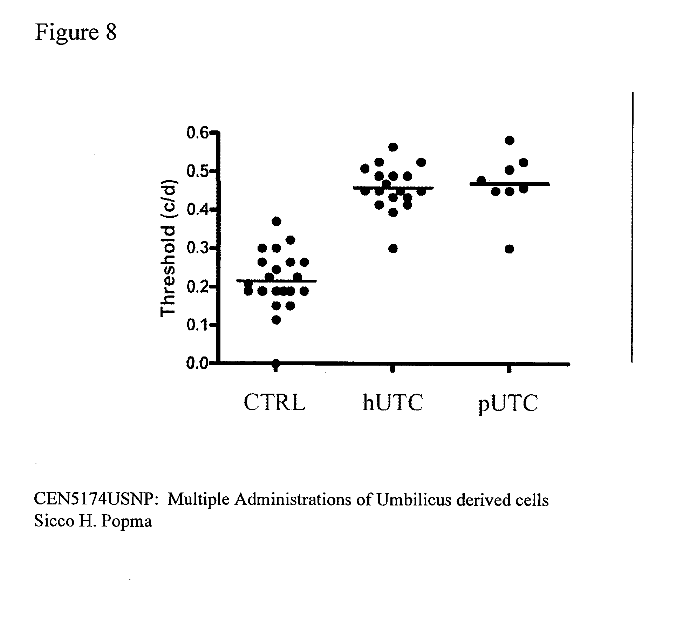

Figure 8 shows optomotor threshold recordings at postnatal day 100 (P100) in RCS rats, comparing the efficacy of human umbilicus-derived cells and porcine umbilicus-derived cells following subretinal administration. UTC (20,000 per rat) were injected subretinally between P20 and P23.

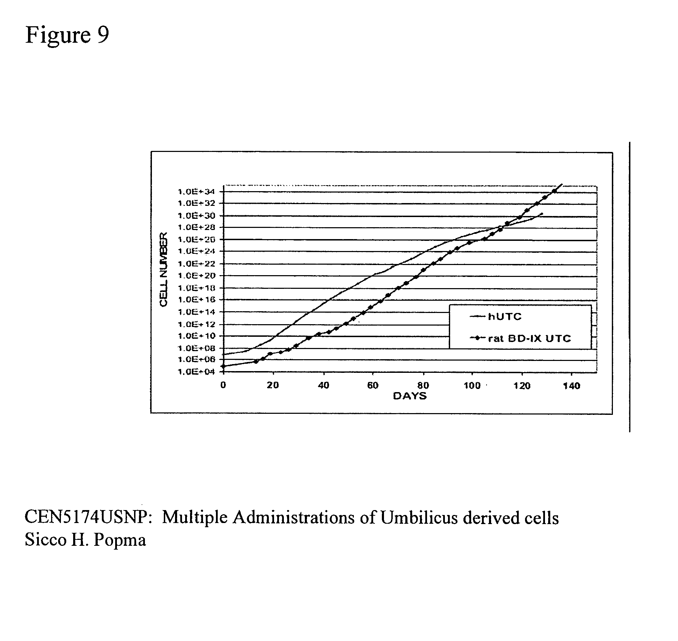

Figure 9 shows the long-term growth curves of human UTC (hUTC) and rat BD-IX UTC. BDIX donor 033105 (dark blue line) is shown below and compared to hUTC (light blue line,). Note that both human and rat UTC populations grow at similar rates as evidenced by their similar slopes. In addition, the morphology of rat UTC was similar to the fibroblastic nature of human UTC (data not shown).

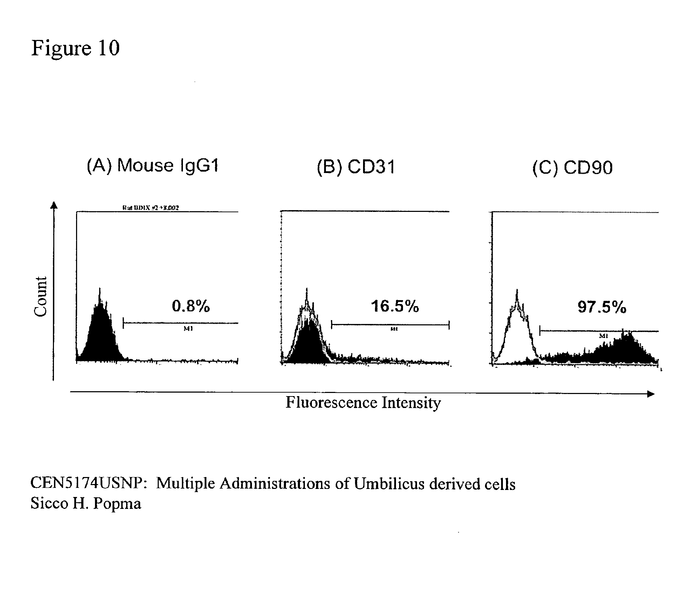

Figure 10 shows the flow cytometry testing the working banks of rat UTC for expression of CD31 (panel B) and CD90 (panel C) compared to the control (panel A).

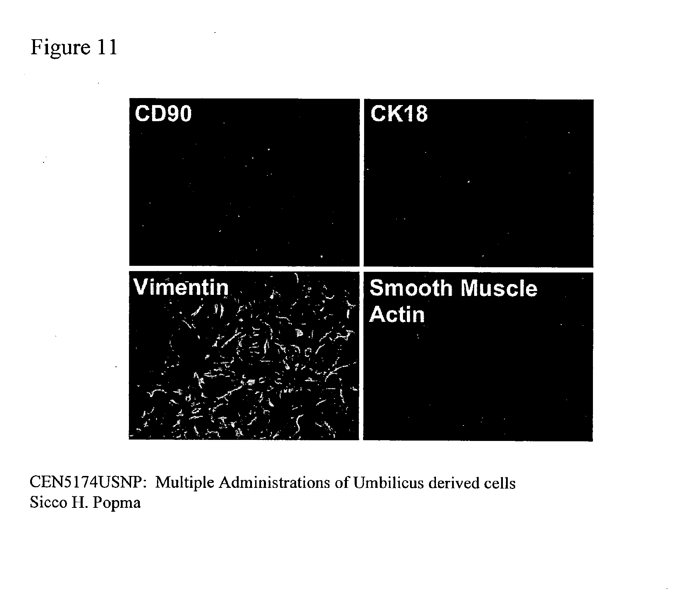

Figure 11 shows the immunocytochemistry results of rat UTC stained for CD90 (upper left), CK18 (upper right), Vimentin (lower left) and smooth muscle actin (lower right).

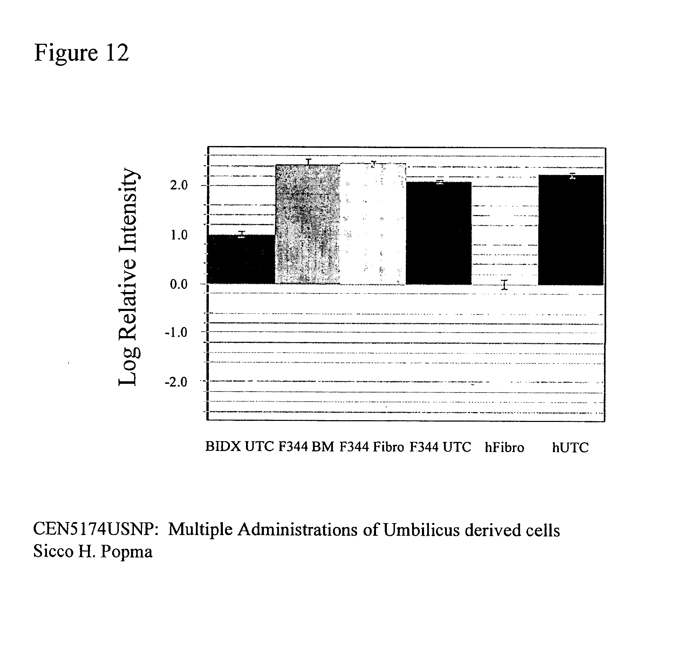

Figure 12 shows the quantitative PCR results for reticulon show relative expression of reticulon in rat BD-IX UTC (pink), Fisher 344 rat bone marrow, fibroblasts, and UTC, and human UTC (hUTC). All data was normalized to control human fibroblasts.

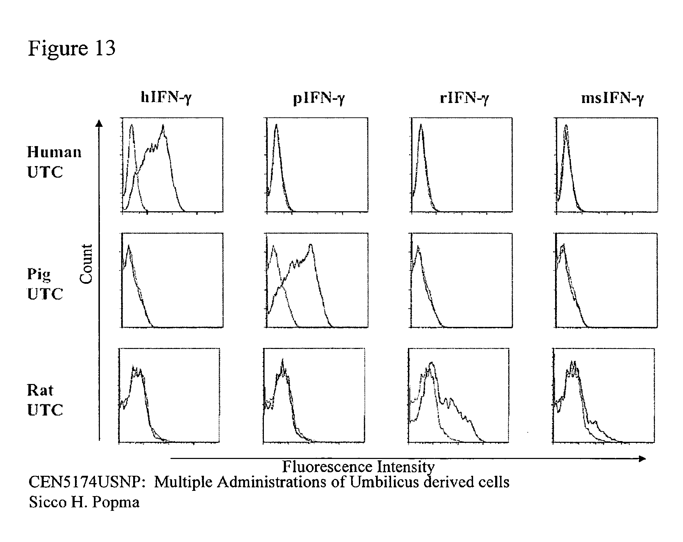

Figure 13 shows the effect of IFN-γ from rat, human and pig on the upregulation of MHC class II (x-axis in each histogram) on umbilicus-tissue derived cells from human (hUTC), pig (pUTC) and rat (rUTC). Grey line is the unstained control and the dark line the sample histogram overlay. It demonstrates that the IFN-γ is species specific (i.e. only human IFN-γ induces the expression of MHC calls II on human UTC).

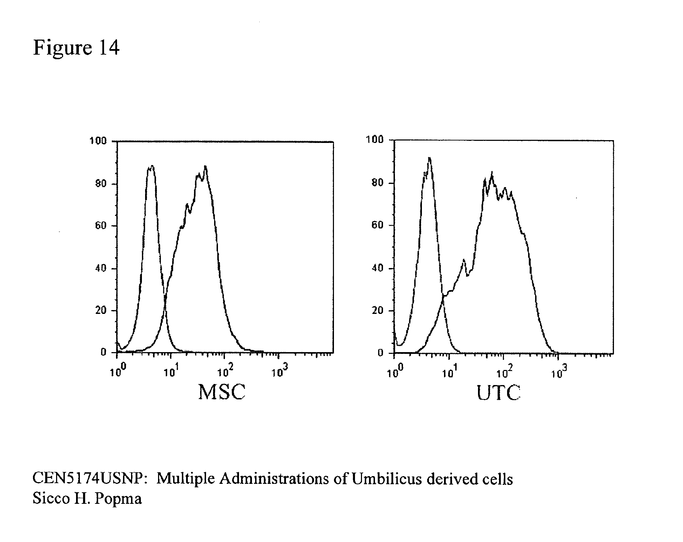

Figure 14 shows the induction of MHC class II expression on both umbilicus-derived cells (right panel) and mesenchymal stem cells (left panel) by IFN-γ. A: Unactivated cells. B: Cells treated with 500U/ml IFN-γ for 48 hrs.

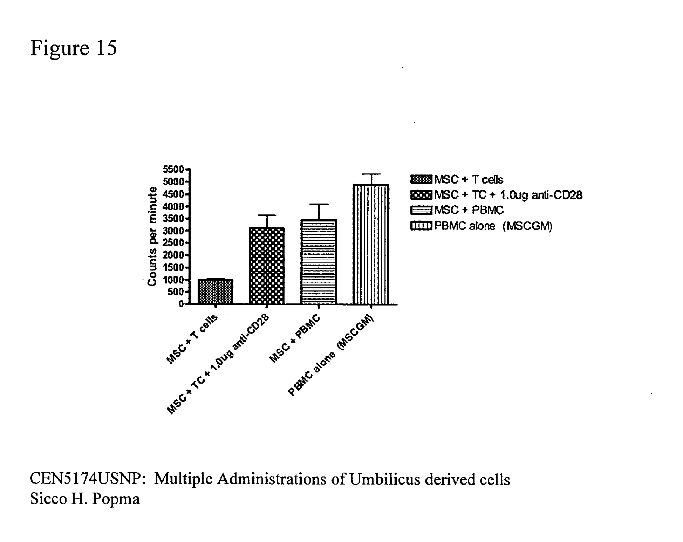

Figure 15 shows proliferation of T cells measured by ³H-Thymidine incorporation (in counts per minute): Purified T cells co-cultured with mesenchymal stem cells (MSC) show little proliferation. PBMC (containing antigen presenting cells) co-cultured with UTC do induce proliferation. Finally, anti CD28⁺ restored the T cell proliferation suggesting that the main function of APC in these cultures is to provide costimulation.

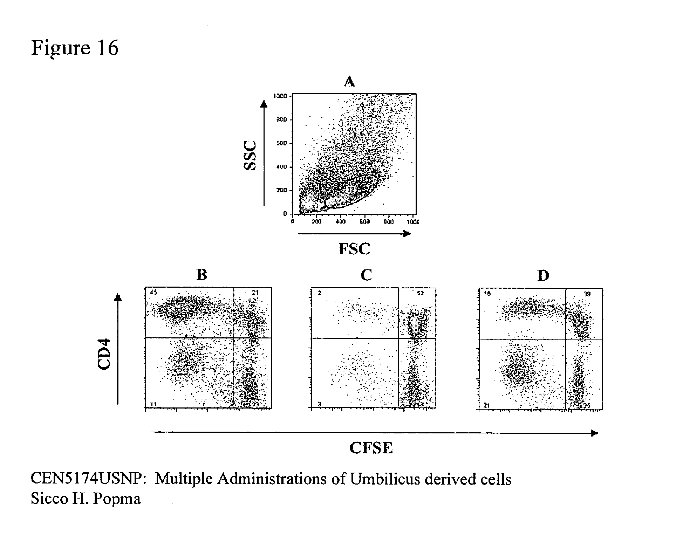

Figure 16 shows that the combination of carboxyfluorescein diacetate succinimidyl ester dilution and flow cytometry allows for a detailed analysis of proliferating cells this in contrast to the ³H thymidine incorporation which measures bulk results. This figure shows T cell proliferation (x-axis), as identified by the T cell marker CD4 (y-axis panel B, C and D) within the viable co-culture cells (panel A), when seeded on human mesenchymal stem cells that have been activated with 500U/ml IFN-γ for 48 hours. B: PBMC: C: T cells: D: T cells + anti-CD28 antibody. As demonstrated in the ³H thymidine incorporation assay, Purified T cells co-cultured with MSC show little proliferation (panel C). PBMC (containing antigen presenting cells) co-cultured with MSC do induce CD4 T cell proliferation (panel B). Finally, anti CD28⁺ restored the T cell proliferation (panel D) suggesting that the main function of APC in these cultures is to provide costimulation.

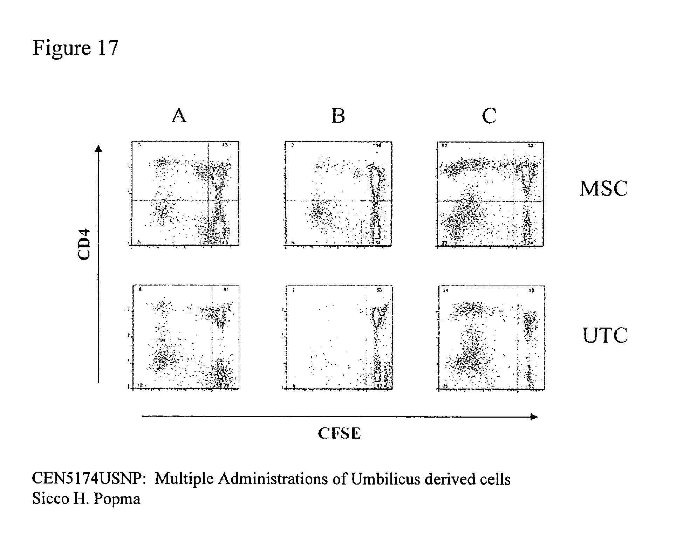

Figure 17 shows the comparison of T cell proliferation co-cultured with human mesenchymal stem cells (MSC) or human umbilicus-derived cells. Flow cytometric analysis of proliferation (x-axis) via carboxyfluorescein diacetate succinimidyl ester dilution showed CD4 T cells (y-axis) proliferated when antigen presenting cells were present either in PBMC (panel A) or when purified antigen presenting cells were added back panel C) and little proliferation in T cells only co-cultures (panel B).

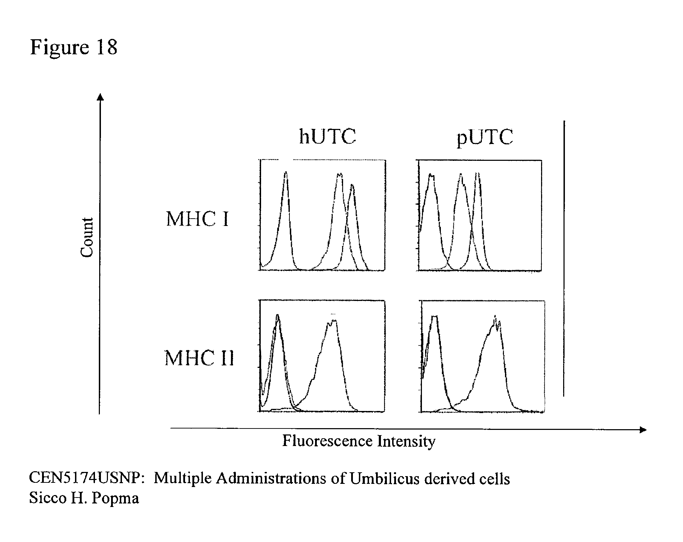

Figure 18 shows that pig IFN-γ induces MHC II expression and upregulates MHC I expression on pig UTC similar to the human UTC. Porcine umbilicus-derived cells flow cytometric analysis for MHC class I (top right panel), or MHC II (bottom right panel) expression. Human umbilicus-derived cells flow cytometric analysis for MHC class I (top left panel) or MHC II (bottom left panel) expression. A: Unstained UTC control: B: Untreated and stained UTC: C: UTC activated with 80ng/ml IFN-γ for 48 hours.

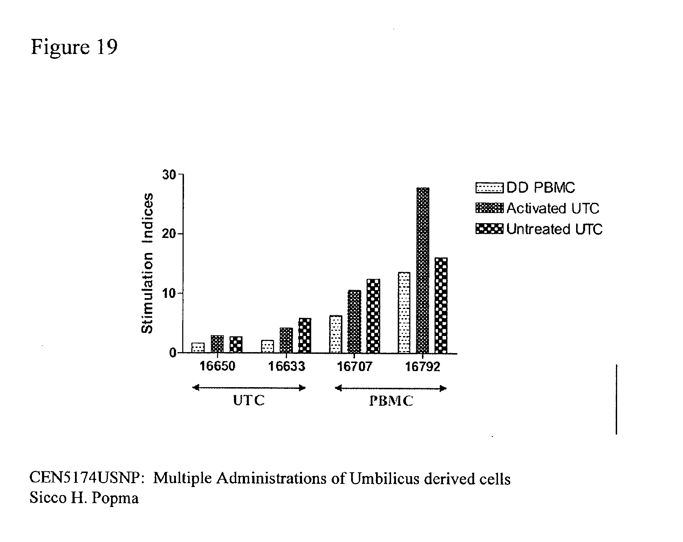

Figure 19 Lymphocyte/UTC co-culture responses measured via ³H-thymidine incorporation show that blood samples isolated from experimental animals injected with porcine umbilicus-derived cells or PBMC (SLA^{dd} haplotype) can be used to determine an adaptive immune response. Y-axis: Stimulation index: sample CPM/back ground CPM, x-axis experimental animals tested (16650 and 16633 were injected SC with UTC and 16707 and 16792 were injected SC with PBMC)

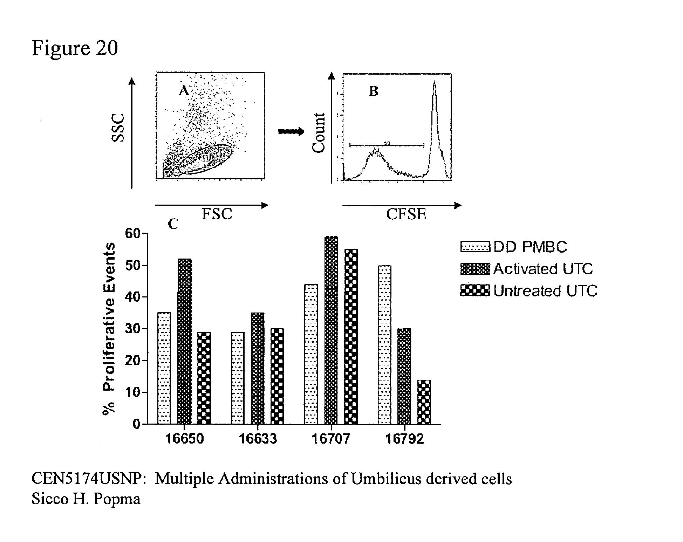

Figure 20 shows assessment of T cell proliferation in co-cultures using flow cytometric analysis and carboxyfluorescein diacetate succinimidyl ester labeling of the responder cells using experimental blood samples. A: selection of the viable cells in the co-cultures (FSC v. SSC gate). B: carboxyfluorescein diacetate succinimidyl ester histogram based on previous gate, gated on proliferating population (diluted carboxyfluorescein diacetate succinimidyl ester stain). C: Graph of % proliferative events based on previous gate per sample. Experimental animals tested: 16650 and 16633 were injected SC with UTC and 16707 and 16792 were injected SC with PBMC.

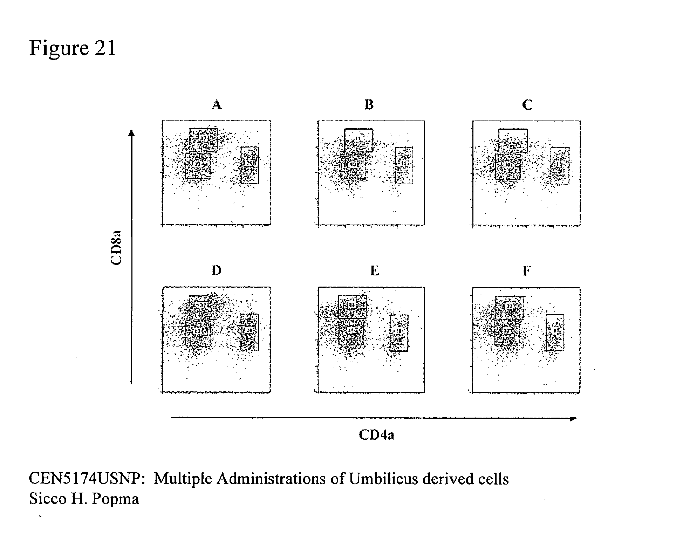

Figure 21 shows the flow cytometry analysis of carboxyfluorescein diacetate succinimidyl ester labeled cells, which allows for detailed analysis of the proliferating cells in MLR. Proliferating cells were selected based on carboxyfluorescein diacetate succinimidyl ester dilution. The less bright carboxyfluorescein diacetate succinimidyl ester labeled cells (proliferated cells) were gated and analyzed for T cell markers. In this example blood samples from a pig injected with UTC SC were tested against: DD PBMC (panel A), AC PBMC (panel B), CC PBMC (panel C), Yucatan PBMC (panel D), Activated DD UTC (panel E) and finally unactivated DD UTC (panel F). The panels show the CD4 (x-axis) vs. CD8 (y-axis) T cells phenotype distribution within the proliferating cell fraction (CD4⁺ T cells are located mid right in each panel and CD8⁺ T cells are located at the upper left in each panel).

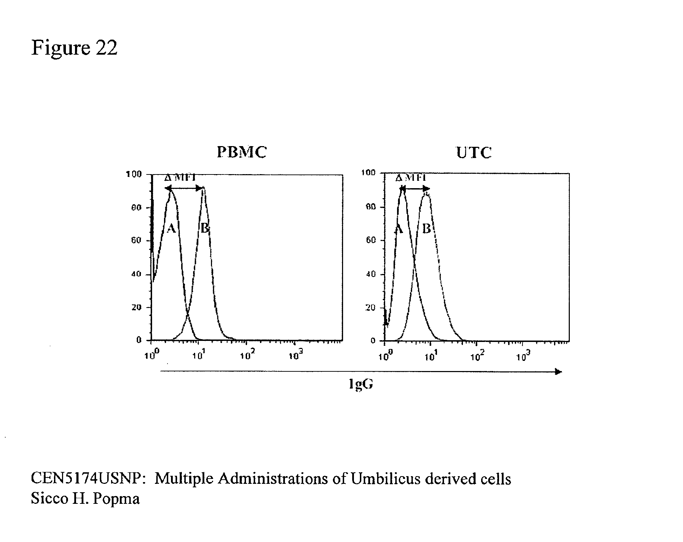

Figure 22 demonstrates that flow cytometry can be used to detect serum antibodies. Serum from animals, sensitized with DD UTC, were incubated with PBMC (left panel) or UTC (right panel) and counterstained with a fluorescent-labeled anti-pig IgG (or IgM, data not shown) antibody and analyzed by flow cytometry. Pretreatment samples (A) did not show a shift in fluorescence whereas the samples obtained after injection of UTC did (B) did, indicating the presence of serum antibodies. (Results shown: animal #16922, injected with activated UTC).

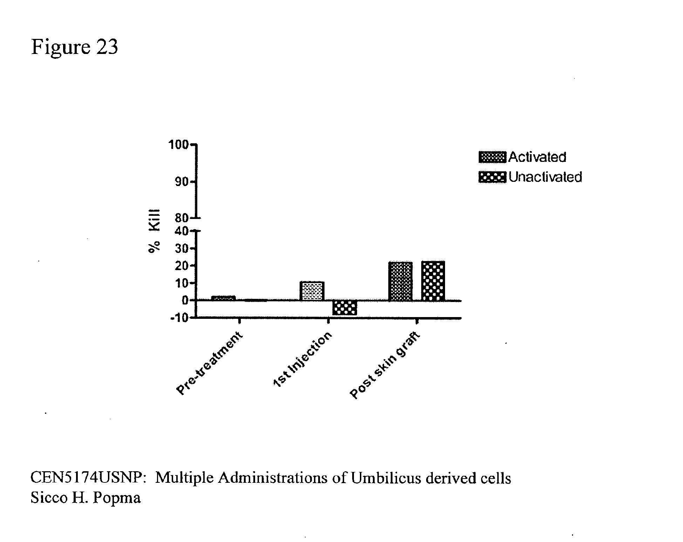

Figure 23 shows that T cell cytotoxicity can be assessed using ACEA-RTCES. PBMC samples were co incubated with UTC grown on E-plates. These plates measure the conductivity of the bottom of each culture well. The conductivity is dependent on the presence of adherent cells (UTC) on the bottom of the well and will decrease when UTC are killed by cytotoxic T cells. There was a change in conductivity when T cells from sensitized animals were co-culture in these plates. As demonstrated in this figure pigs that were sensitized with allogeneic DD skin grafts showed the presence of Cytotoxic T cells with the capability to kill the UTC after 4 hour of co-culture (y-axis % kill, x-axis assessment time points).

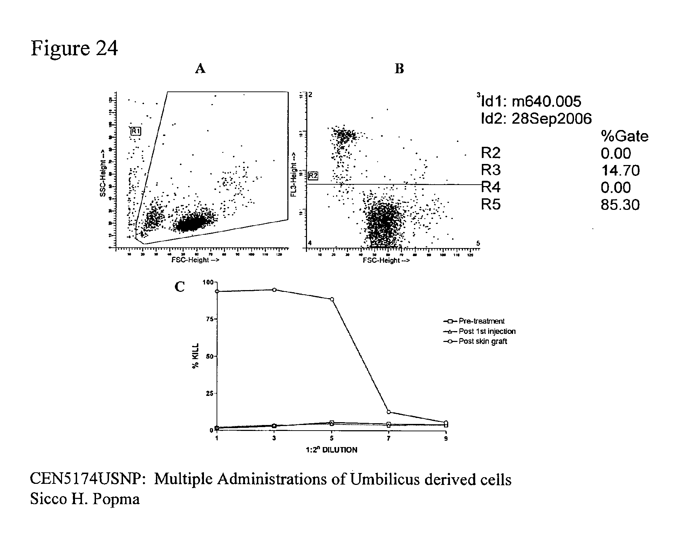

Figure 24 Assessment whether serum antibodies are cytotoxic using dye exclusion flow cytometry. Target cells (DD PBMC) were incubated with serum samples obtained from experimental animals and rabbit complement was added. If the antibodies were cytotoxic it would perforate the cell membrane of the target cells (panel A) and allow a fluorescent dye (7-AAD) to permeate the cell (top half panel b). Viable cells will not be stained by the dye (bottom half panel B). The ratio of viable versus dead cells determines the percent kill (y-axis, panel C).

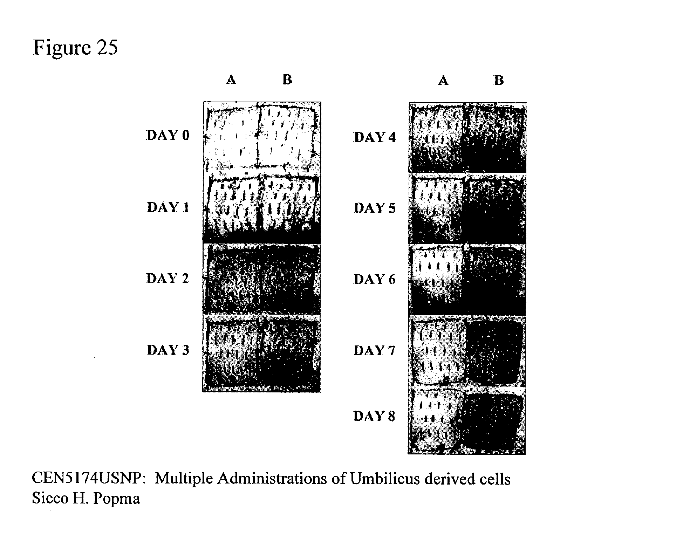

Figure 25 demonstrates the utility of skin grafting in this model. The photographs depict the appearance of the skin grafts. This series of photographs recorded a normal rejection of a disparate skin graft (DD, column B) compared to the acceptance of an auto graft (CC, column A) over a period of 8 days.

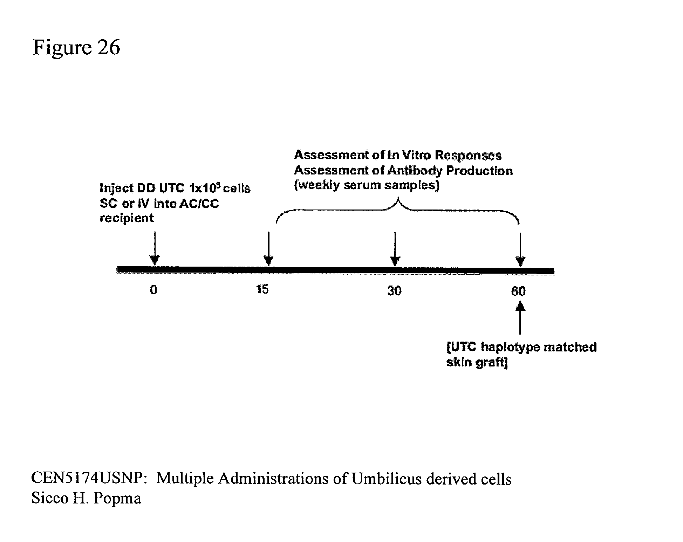

Figure 26 shows the experimental protocol for the immune response assessment after single injection of "DD" UTC into "AC" or "CC" miniswine

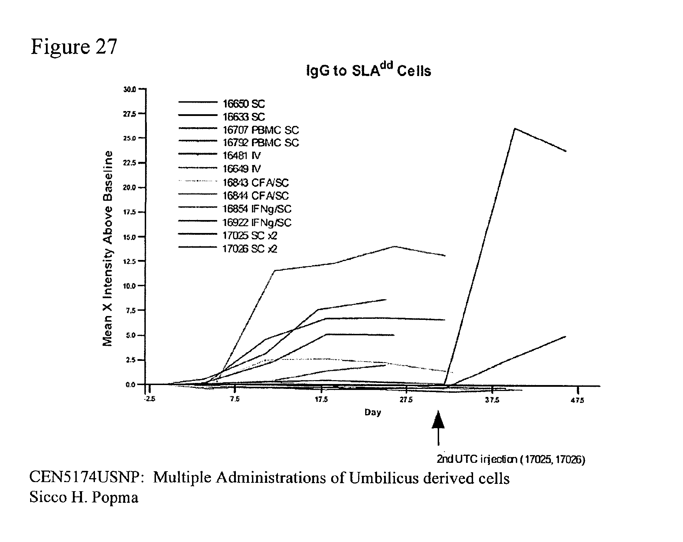

Figure 27 shows the results of the serum antibody detection assays for the experimental animals. Samples were incubated with SLA^{dd} PBMC and serum binding was detected with a fluorescent secondary antibody. The mean fluorescence signal shift was calculated (y-axis, signal sample-signal control) using experimental samples acquired over time. This figure shows that PBMC, activated UTC (IFN-γ/SC), UTC injected into an inflamed area (CFA/SC) and multiple subcutaneous injections lead to a serum antibody response whereas subcutaneous UTC do not induce a measurable antibody response.

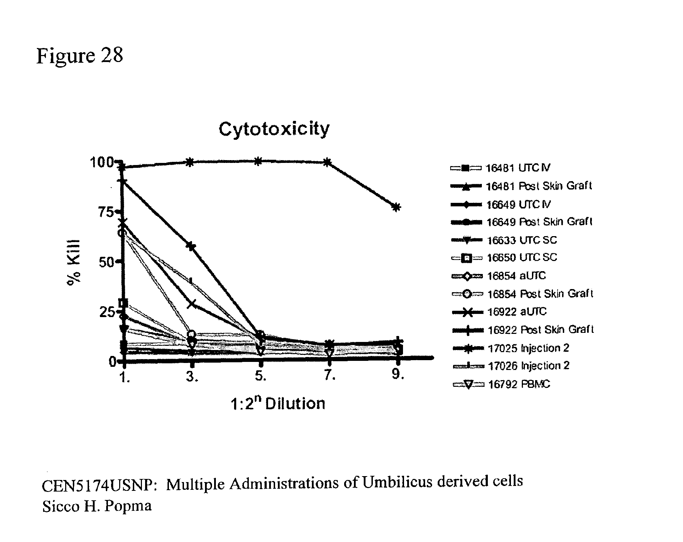

Figure 28 shows the summary results for cytotoxic antibody detection using flow cytometry. Significant lysis of the target cells by cytotoxic serum antibodies was detected after skin graft (16854 and 16922), second injection (17025 and 17026) and in one out of the two pigs injected with the activated UTC (16922). This indicates that these animals have developed an adaptive immune response whereas the remainder of the test animals did not demonstrate a detectable adaptive immune response in this assay

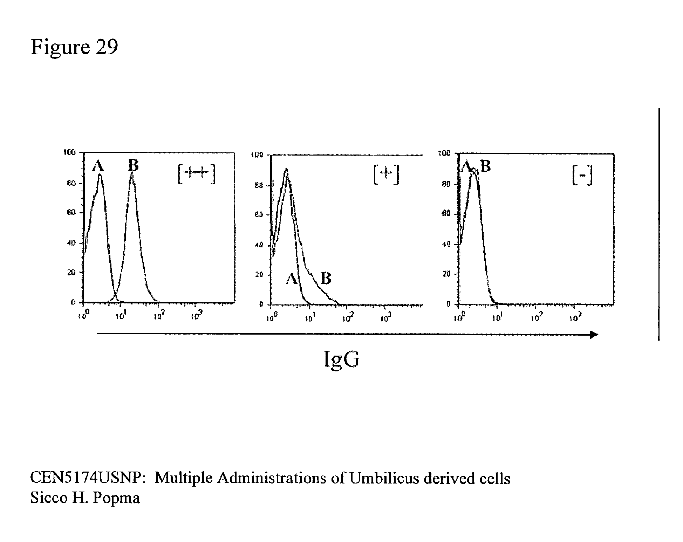

Figure 29 shows that anti UTC serum antibody (IgG) are mainly directed against MHC I. Shown is the binding to cells with the D haplotype for class I and class II ("DD" left panel), binding to cells that share the MHC II haplotype ("CD", middle panel) and the binding for serum obtained at the time of skin graft ("CD", right panel).

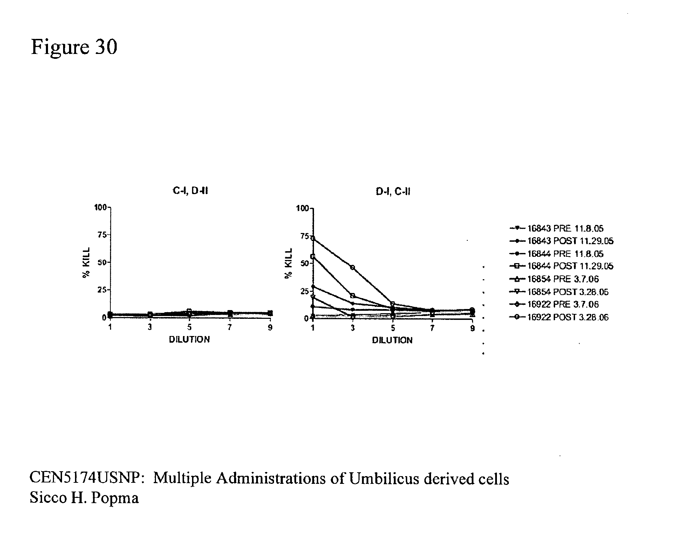

Figure 30 shows that the Class I of "D" MHC molecules induces a T cell response that is cytotoxic and that anti "DD" serum antibodies causes preferential lysis of targets expressing "D" of class I and not "D" of class II

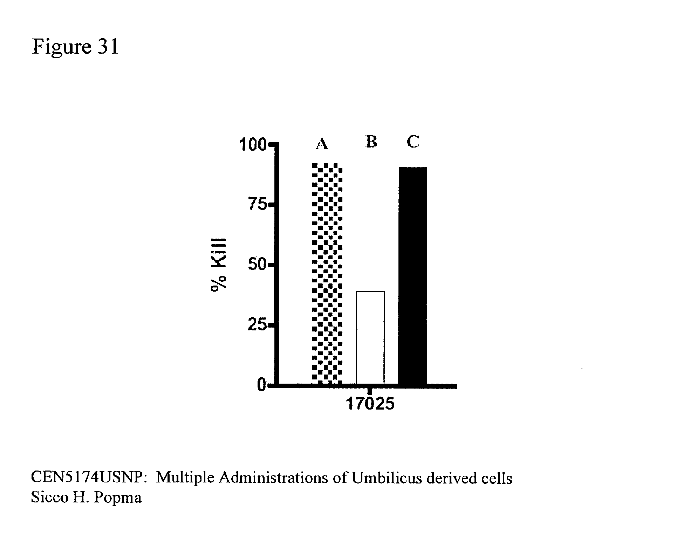

Figure 31 shows that cytotoxic T cell responses are significantly enhanced using the ACEA assay when the experimental blood samples are restimulated in vitro with cells mismatched for MHC I ("DC", column C) than MHC II ("CD", column B). The MHC I mismatch demonstrates as much kill as the full MHC I and MHC II mismatch ("DD"). This suggest that the MHC I molecule it the main target of the cytotoxic T cells, i.e. the cytotoxic T cells kill cells expressing MHC I of "D".

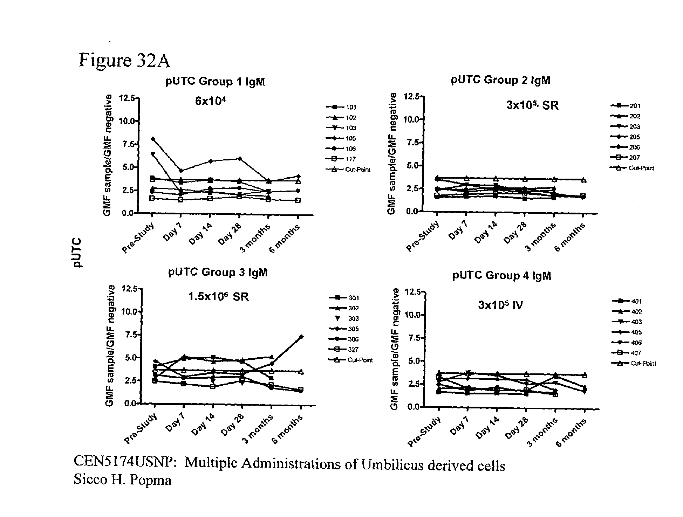

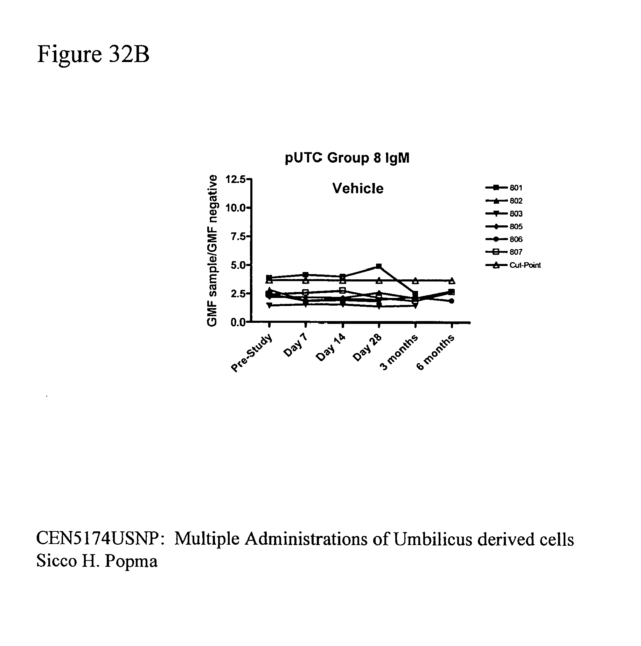

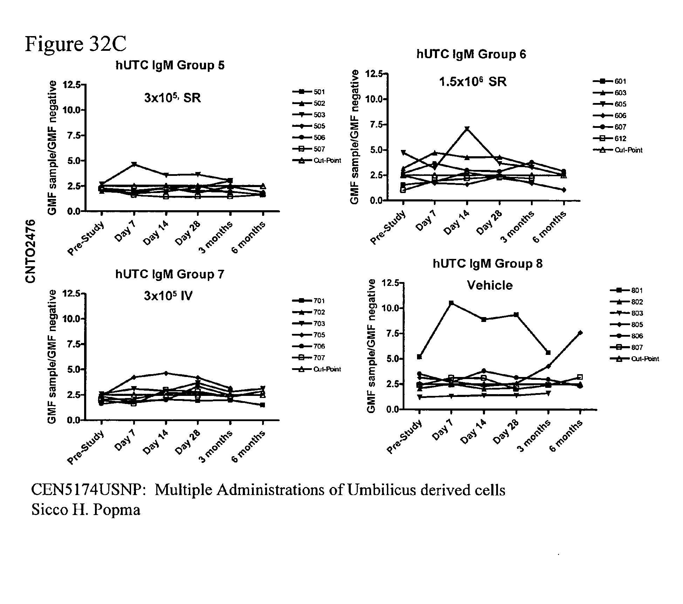

Figure 32 shows IgM levels in serum samples obtained from animals that were either subretinally injected with pUTC or hUTC (CNTO2476). The graphs show the serum antibody response over time (pre treatment, day 7, day 14 day 28, 3 month and 6 months) per treatment group. The y-axis shows the mean difference in fluorescence compared to control and the x-axis the time points. There were IgM responses recorded however there were IgM responses in the control group also, which make the interpretation of the IgM results difficult.

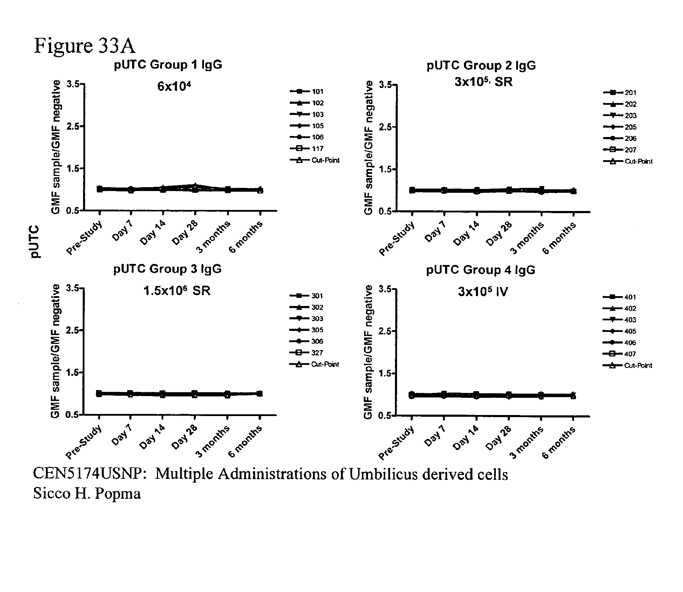

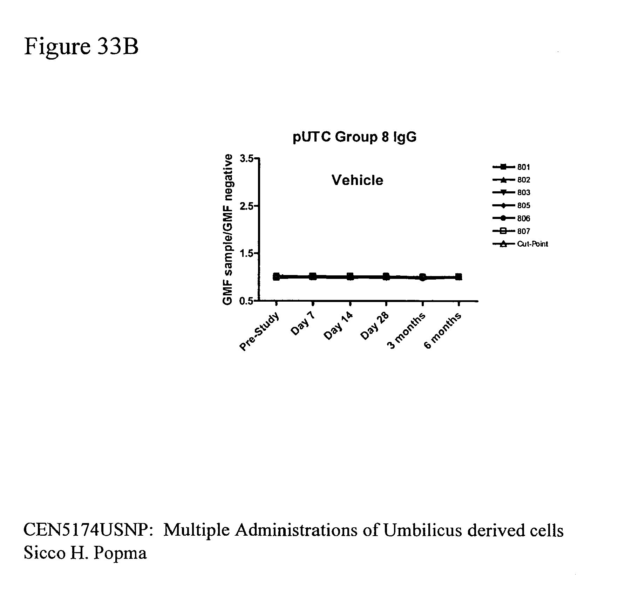

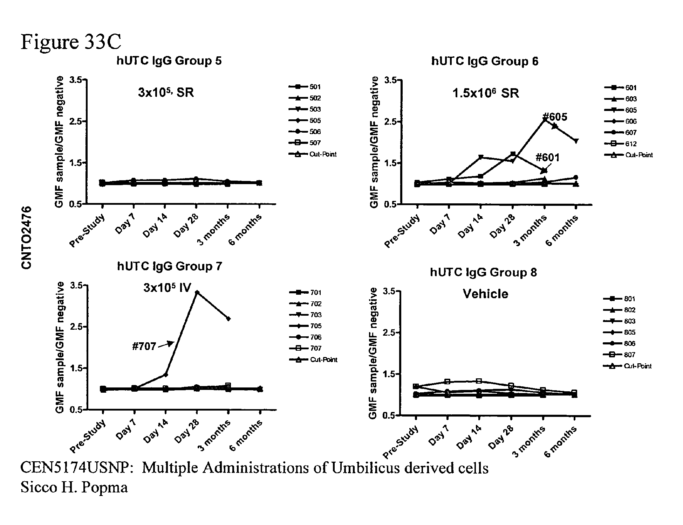

Figure 33 shows IgG levels in serum samples obtained from animals that were either subretinally injected with pUTC or hUTC (CNTO2476). The graphs show the serum antibody response over time (pre treatment, day 7, day 14 day 28, 3 month and 6 months) per treatment group. The y-axis shows the mean difference in fluorescence compared to control and the x-axis the time points. Animals 601, 605 and 705 showed clear IgG responses to xenogeneic hUTC. No animals demonstrated a measurable IgG response to allogeneic pUTC.

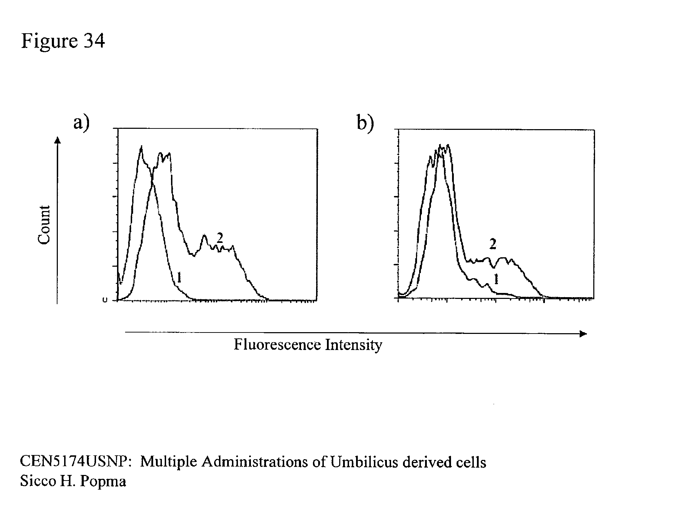

Figure 34 shows that rat IFN-γ induces MHC class II upregulation on rat UTC. The left panel shows the MHC class II upregulation by 50U of IFN-γ for 72 hours (1=unactivated control, 2=activated sample). The right panel shows MHC class II upregulation after 48h with 500U IFN-γ (1=unactivated control, 2=activated sample)

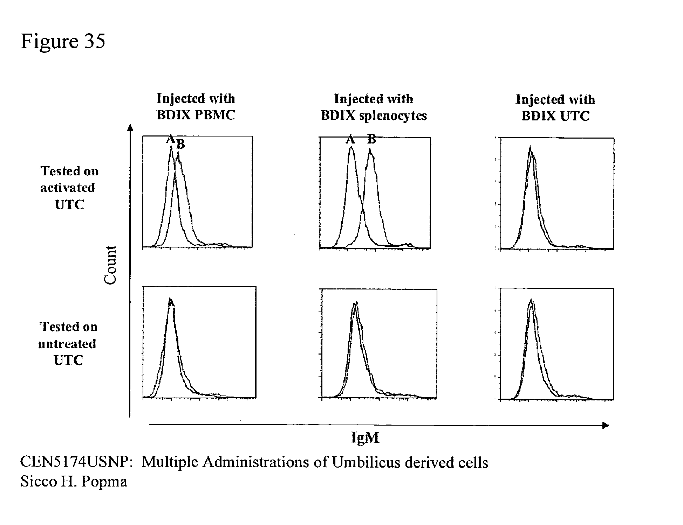

Figure 35 shows IgM levels in serum samples obtained from of rats 21 days after being injected with BDIX PBMC, BDIX splenocytes, or BDIX UTC. The plots show the control.

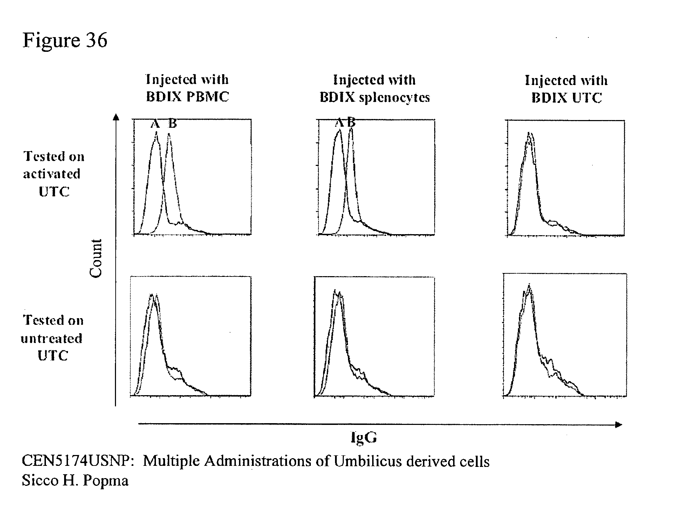

Figure 36 shows IgG levels in serum samples obtained from of rats 21 days after being injected with BDIX PBMC, BDIX splenocytes, or BDIX UTC. The plots show the control histograms (A or red) overlayed with the sample histograms (B or green). Serum obtained from animals injected SC with allogeneic PBMC or splenocytes bound activated (MHC I and MHC II expressing) UTC.

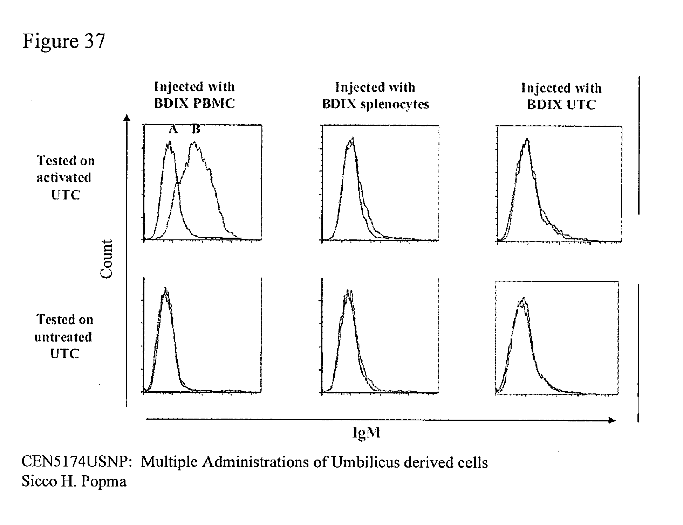

Figure 37 shows IgM levels in serum samples obtained from of rats 28 days after being injected with BDIX PBMC, BDIX splenocytes, or BDIX UTC. The plots show the control histograms (A or red) overlayed with the sample histograms (B or green). Serum obtained from animals injected SC with allogeneic PBMC or splenocytes bound activated (MHC I and MHC II expressing) UTC strongly.

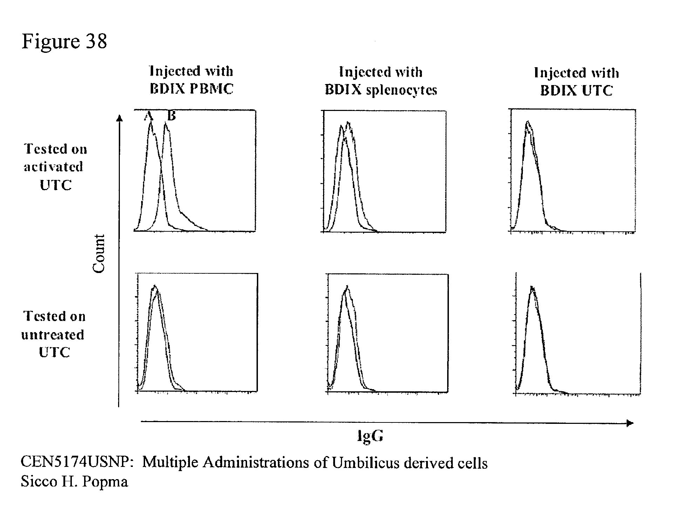

Figure 38 shows IgG levels in serum samples obtained from of rats 28 days after being injected with BDIX PBMC, BDIX splenocytes, or BDIX UTC. The plots show the control histograms (A or red) overlayed with the sample histograms (B or green). Serum obtained from animals injected SC with allogeneic PBMC or splenocytes bound activated (MHC I and MHC II expressing) UTC.

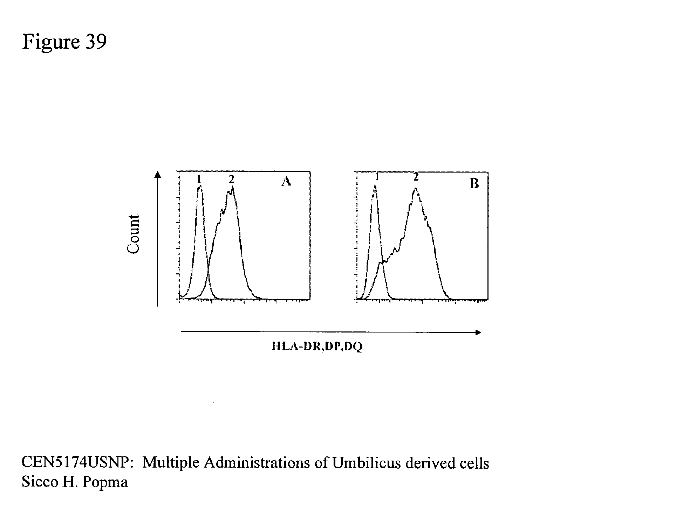

Figure 39 shows MHC class II expression on MSC and UTC was verified before the cells were injected into the test animals. MHC was upregulated on both MSC (panel A) and UTC (panel B) as demonstrated in the overlays of unactivated cells (1) and activated cells (2).

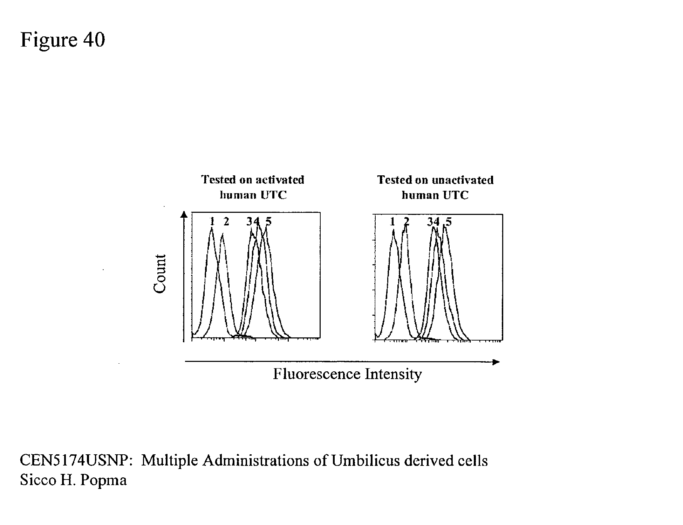

Figure 40 shows the IgG serum antibody of rats injected with human UTC. Serum samples were collected pretreatment (#1), day 7 (#2), day 14 (#3), day 21 (#4) and day 28 (#5) and the binding to activated UTC (left panel) and unactivated UTC (right panel) was assessed.

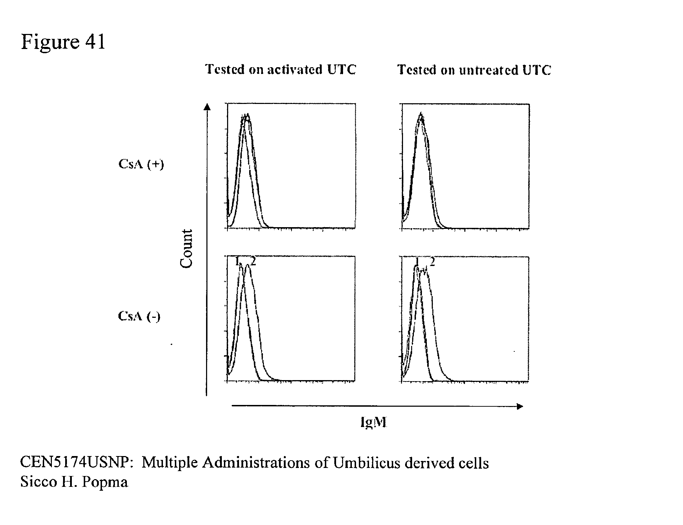

Figure 41 shows the effect of cyclosporine A (CsA) on IgM levels in serum of rats that have been treated with human umbilicus-derived cells. Serum of untreated animals (bottom panels) staining either activated (bottom left) or unactivated (bottom right) UTC. Results for CsA treated animals are shown in the top panels.

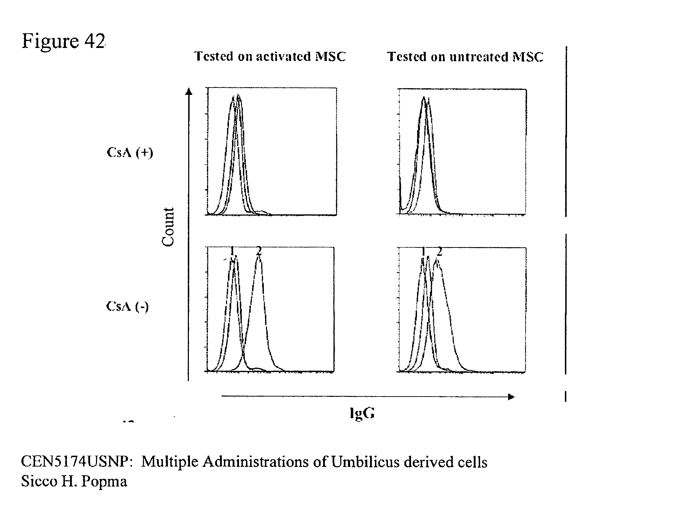

Figure 42 shows the effect of cyclosporine A (CsA) on IgG levels in serum of rats that have been treated with human mesenchymal stem cells. Serum results of untreated animals (bottom panels) binding to either activated (bottom left) or unactivated (bottom right) UTC. Results for CsA treated animals are shown in the top panels.

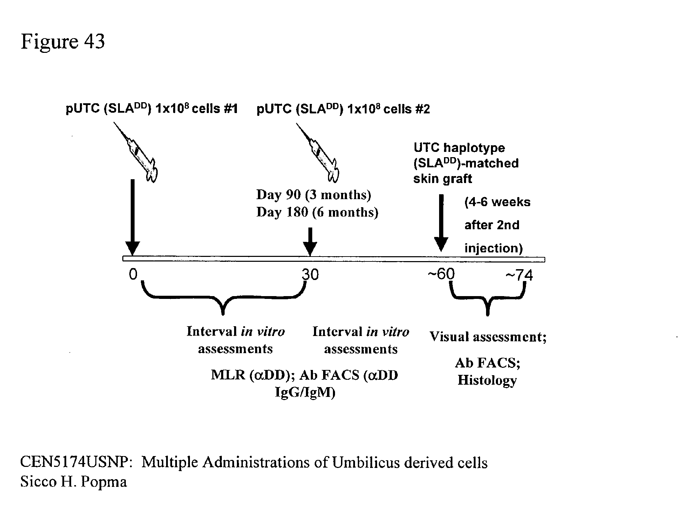

Figure 43 shows the general study outline regarding injection and skin graft schedule and sampling time points at which blood and serum samples were collected.

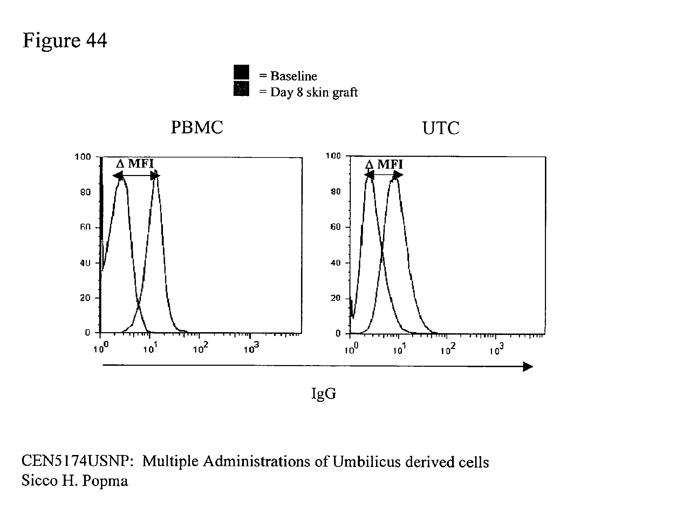

Figure 44 shows the analysis of serum from sensitized animal post skin graft with IgG directed against DD PBMC or DD UTC compared to serum from a naïve animal before UTC injection (pre-treatment), as measured by the shift in mean fluorescence intensity.

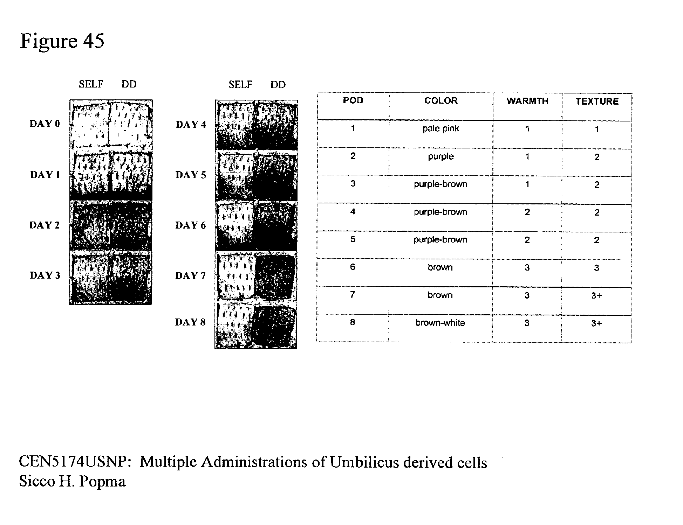

Figure 45 shows skin grafts of animals enrolled in the studies that showed antibodies directed against DD PBMC received CC (self) and DD skin grafts. Graft rejection was assessed for 8 days by color, warmth (1=warm, 2=less warm, 3-cool, cooler), and texture (1=soft, 2=less soft, slightly hard, 3=firm, hard) to determine rejection of grafts. The photographs and table shown an example of the normal rejection process of mismatched skin and survival of matched (self) skin.

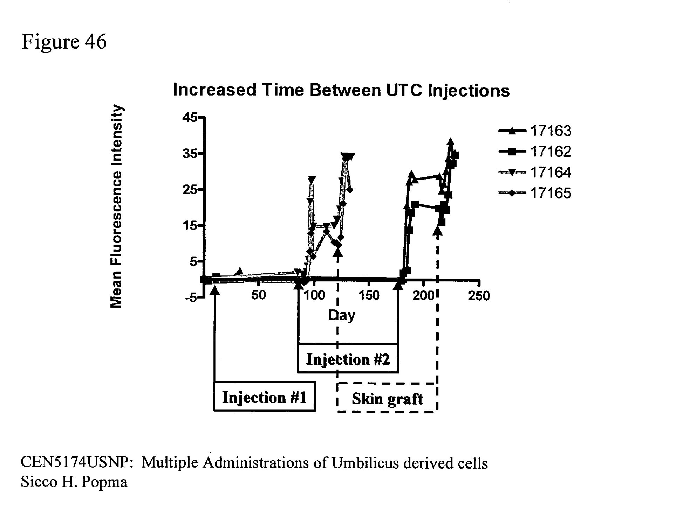

Figure 46 shows serum antibody analysis results for the repeat injections after 3-month (17164 and 17165) or 6-month (17162 and 17163).

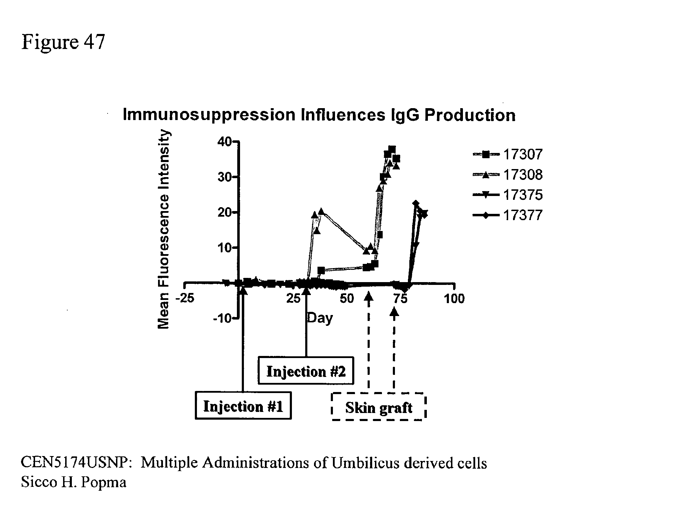

Figure 47 shows serum IgG antibody results of animals treated with CsA and prednisone at each injection (17375 and 17377). Results for treatment with prednisone administered at the second injection only (17307 and 17308.).

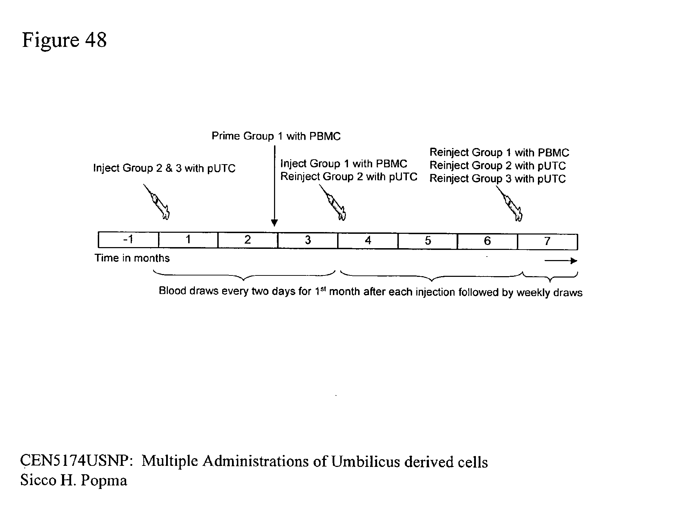

Figure 48 shows the general study outline regarding injection and sampling time points at which blood and serum samples were collected.

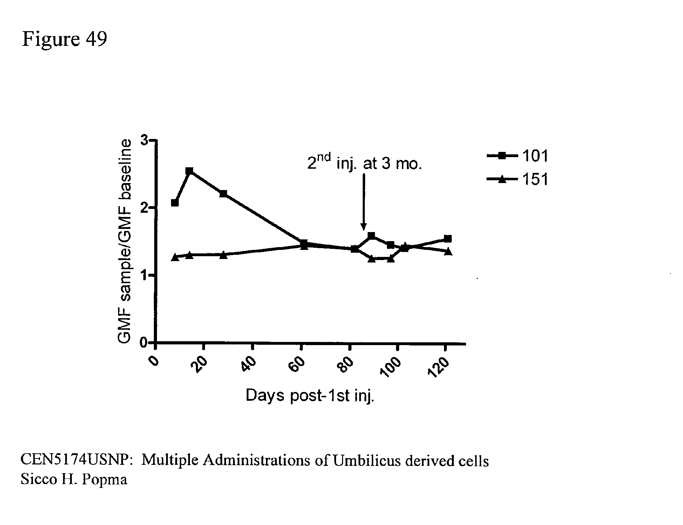

Figure 49 shows serum IgG antibody results from two animals (animals # 101 and 151) that received two subretinal injections of PBMC spaced 3 months apart after they were sensitized with subcutaneous injections of PBMC, which were matched to the SR injected UTC.

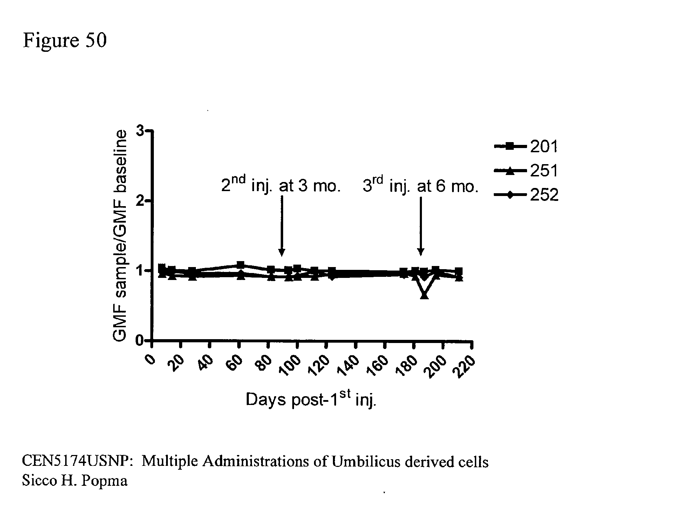

Figure 50 shows serum IgG antibody results of animals receiving repeat subretinal injections ofpUTC at 3 months and again at 6 months (animal # 201, 251 and 252).

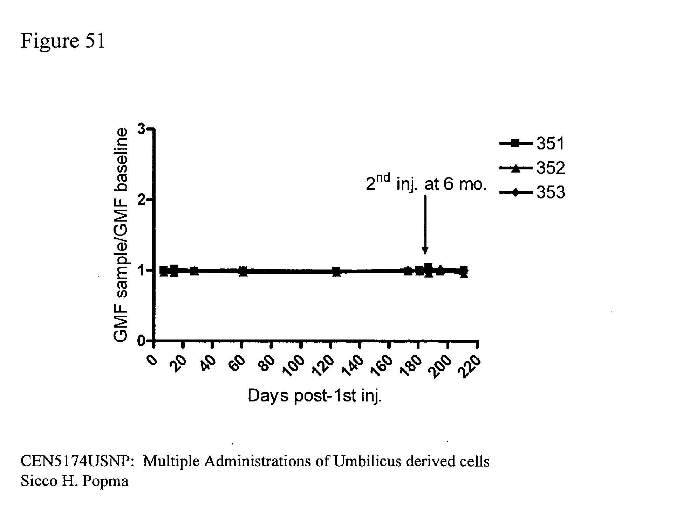

Figure 51 shows serum IgG antibody results of animals receiving repeat subretinal injections of pUTC 6-months after the first injection (animal # 351, 352 and 353).

### DETAILED DESCRIPTION

For clarity of disclosure, and not by way of limitation, the detailed description of the invention is divided into the following subsections that describe or illustrate certain features, embodiments, or applications of the present invention.

In one embodiment, the present invention provides methods to determine the optimal administration protocol of allogeneic donor tissue to treat a disease in a human, using an animal model of human disease, comprising the steps of:
a. Obtaining allogeneic donor tissue from a donor animal,
b. Inducing a disease state in a recipient animal,
c. Administering the allogeneic donor tissue to the recipient animal, and monitoring the immune response elicited by the allogeneic donor tissue, and
d. Altering the administration of the allogeneic donor tissue to reduce the immune response in the recipient animal.

In one embodiment, the present invention provides methods to determine the optimal administration protocol of allogeneic donor tissue to treat a disease in a human, using an animal model of human disease, comprising the steps of:
a. Obtaining allogeneic donor tissue from a donor animal,
b. Inducing a disease state in a recipient animal,
c. Administering the allogeneic donor tissue to the recipient animal, and monitoring the immune response elicited by the allogeneic donor tissue,
d. Monitoring the efficacy of the allogeneic donor tissue in the recipient animal, and
e. Altering the administration of the allogeneic donor tissue to reduce the immune response in the recipient animal.

The present invention may utilize any animal model of human disease. The accuracy of the prediction of the immune response in a human recipient may depend on factors, such as, for example, the similarities between the animal and human donor tissue, and similarities between the animal and human immune response. In one embodiment, the immune response of the animal recipient reflects that of the human recipient.

The similarity of the animal and human allogeneic donor tissue may be determined by any method in the art. For example, the biological properties between the animal and the human allogeneic donor tissue may be determined. Similarities in protein secretion, cell membrane composition, and cell surface marker expression are all examples of the parameters that may be used to determine the similarity of the allogeneic donor tissue. Examples 1-3, and 5-11 of the present invention describe methods whereby the properties of rat, human and pig umbilicus-derived cells are compared *in vitro* and *in vivo.*

The allogeneic donor tissue may be a solid organ, such as, for example, a heart. Alternatively, the allogeneic donor tissue may comprise cells. For example, the cells may be mesenchymal stem cells. Alternatively, the cells may be cells derived from umbilical cord. In one example, the allogeneic donor tissue is umbilicus-derived cells.

In one embodiment of the present invention, allogeneic umbilicus-derived cells are administered into an animal model of human disease to determine the optimal administration protocol for administering allogeneic umbilicus-derived cells into a human suffering from that disease.

### The method

The present invention utilizes an animal model of human disease to refine the administration of allogeneic donor tissue to optimize efficacy, while minimizing the immune response elicited by the allogeneic donor tissue. A series of iterative experiments are contemplated in this optimization process, where the results and observations of a preceding experiment are used to alter the parameters of a subsequent experiment, such that an optimal administration protocol is designed. Observations may include, for example, the survival of the allogeneic donor tissue in the recipient animal, the efficacy of the allogeneic donor tissue, the immune responses observed.

The number of experiments in a series may depend a number of parameters, including, for example, the animal model, the nature of the disease, the allogeneic donor tissue, and the like. The optimal administration protocol may be determined using one animal, or more than one animal may be required.

The optimal site of administration, the optimal time of the first administration after onset of the disease, the optimal amount of donor tissue administered, and the optimal number of administrations may all be determined using the method of the present invention.

The effect of treating the recipient with least agent that decreases the immune response may be determined. Alternatively, the effect of treating the allogeneic donor tissue with least agent that decreases the immune response may also be determined.

In one embodiment, the at least one agent that reduces the immune response is an anti-inflammatory compound, such as, for example compounds disclosed in U.S. Patent 6,509,369.

In one embodiment, the at least one agent that reduces the immune response is an immunosuppressive compound, such as, for example, compounds disclosed in U.S. Published Application 2004/0171623.

Examples 4 and 12-18 of the present invention show experiments where the administration of umbilicus-derived cells is optimized for the treatment of a variety of diseases, using the methods of the present invention.

### Administration of the allogeneic donor tissue

Allogeneic donor tissue, comprising cells may be administered into the recipient by a variety of methods. The choice of method depends factors, such as, for example, on the disease being treated, the nature of the allogeneic donor tissue, the immune status of the recipient, and the like. The allogeneic donor tissue may be infused into the patient. The allogeneic donor tissue may be injected into the patient at various sites. The allogeneic donor tissue may be surgically implanted. The allogeneic donor tissue may be implanted as cells in suspension, or the cells may be aggregated, or the cells may be incorporated in solid supports or encapsulating materials. The site of administration may be a site where the donor tissue is required. Alternatively, the site of administration may be a site removed from where the donor tissue is required.

The amount of allogeneic donor tissue administered depends on factors such as, for example, the amount sufficient to treat the disease, an amount sufficient to survive in the recipient, and the response of the recipient's immune system to the allogeneic donor tissue.

Examples of methods for the use of allogeneic cells to treat ophthalmic diseases and disorders may be found in US20060280729 and US20060234376.

Examples of methods for the use of allogeneic cells to treat neurodegenerative diseases may be found in US20060233766 and US20060233765.

Examples of methods for the use of allogeneic cells to treat diseases of the heart or circulatory system may be found in US20060188983.

Examples of methods for the use of allogeneic cells to treat diseases of the bone and cartilage may be found in US20060154367.

### Animal models of human disease

Examples of animal models for ischemic injuries may be found in Dib N, et al. J Pharmacol Toxicol Methods. 2006 May-Jun;53(3):256-63, Makkar RR et al. J Cardiovasc Pharmacol Ther. 2005 Dec;10(4):225-33, Hiratsuka K et al. J Surg Res. 2000 Aug;92(2):250-4, and Kim BO et al. Circulation. 2005 Aug 30;112(9 Suppl):I96-104.

Examples of animal models for soft tissue injuries may be found in Norman H, et al. Acta Anaesthesiol Scand. 2006 Oct;50(9):1058-67, Gallant-Behm CL, Hart DA. Wound Repair Regen. 2006 Jan-Feb;14(1):46-54, and Bordais A, et al. Neuromuscul Disord. 2005 Jul;15(7):476-87.

Examples of animal models for bone or connective tissue injuries may be found in Tanaka E, et al. J Orofac Pain. 2005 Fall;19(4):331-6, and Marx RE. Int J Oral Maxillofac Implants. 2006 Mar-Apr;21(2):190-1.

Examples of animal models for diabetes may be found in Hu X, et al. Acta Biochim Biophys Sin (Shanghai). 2007 Feb;39(2):131-6, Suzuma I, et al. Hypertension. 2007 Feb;49(2):347-54, Rogers SA, et al. Transpl Immunol. 2006 Nov; 16(3-4): 176-84, Brandhorst D, et al. Cell Transplant. 2006;15(4):311-7, and Koopmans SJ, et al. Metabolism. 2006 Jul;55(7):960-71.

Animal models of human disease continue to be developed and are contemplated for use according to the methods of the present invention.

Examples of methods for the use of allogeneic cells to treat diseases of the bone and cartilage may be found in US20060154367.

### Detecting the immune response in vitro

The immune response elicited by the donor tissue may be monitored *in vitro* using a cytotoxic T cell response assay. Examples of this assay are disclosed in Glamann J and Hansen AJ Assay Drug Dev Technol. 2006 Oct;4(5):555-63; and Zhu J, et al, Immunol Methods. 2006 Feb 20;309(1-2):25.

The immune response elicited by the donor tissue may be monitored *in vitro* using a serum antibody detection assay. The assay used was a modification on the techniques described by for example Wong BS et al. Transplantation. 2006 Aug 15;82(3):314-9.

The immune response elicited by the donor tissue may be monitored *in vitro* using a cytotoxic serum antibody detection assay. The assay used was a modification of the techniques described by for example Diaz TM, et al. Transplant Proc. 2003 Aug;35(5):2047-8.

### Detecting the immune response in vivo

In one aspect of the present invention, the immune response elicited by the donor tissue may be monitored *in vivo* using the inbred miniature swine disclosed in US20030115620. The miniature swine have been purposely bred to contain several known MHC haplotypes to facilitate transplantation research in a large animal model. In one aspect of the present invention, umbilicus derived cells were derived from umbilicus tissue obtained from the miniature swine, and were administered to the animals.

The immune response elicited by the donor tissue may be monitored *in vivo* using skin grafts, such as, for example, as disclosed in Rosengard, BR Transplantation. 1991 Dec;52(6):1044-52.

The present invention is further illustrated, but not limited by, the following examples.

### Example 1

### Umbilicus-derived cells obtained from inbred MHC-defined miniature swine

*Source of umbilical tissue and Herd Qualification:* Porcine umbilical cords, from miniature swine, were obtained from via cesarean section approximately 2-5 days prior to full-term. This colony, the Massachusetts General Hospital (MGH) Miniature Swine Colony under the general direction of Dr. David Sachs (Transplantation Biology Research Center, MGH, Boston, MA), is specifically characterized at the Swine Leukocyte Antigen locus (SLA). In this study, cords were harvested from SLAdd (class I d/d, class II d/d) pregnant pigs. The miniature swine colony is described in US20030115620.

Following cesarean section, cords were immediately transferred into sterile Dulbecco's modified Eagle's Medium (DMEM) containing 4.5 g/l glucose (Mediatech, Inc., Herndon, VA) for overnight transport on ice to SCIV research labs (Radnor, PA). Four cords in total were harvested and designated DD1, DD2, DD3, and DD4.

*Umbilical tissue processing and initial culture of the porcine umbilicus-derived cells:* The following process was repeated for each cord. The cord was washed in phosphate-buffered saline (PBS; Invitrogen, Carlsbad, CA) to remove blood and debris. The tissue was then mechanically dissociated in 500 cm² tissue culture plates using scalpels until the tissue was minced into a fine pulp. Chopped tissue was then transferred to 50 ml conical tubes containing a mixture of the enzymes collagenase (10 U/ml, Sigma, St. Louis, MO), dispase (12.6 U/ml, Roche Diagnostics, Indianapolis, IN), and hyaluronidase (1 U/ml, Vitrase®, ISTA Pharmaceuticals, Irvine, CA) diluted in DMEM-low glucose medium (Invitrogen, Carlsbad, CA). Tubes were incubated at 37° C in an orbital shaker (Barnstead Lab Line, Melrose Park, IL) at 225 rpm for 2 hours.

After digestion, the tissue was centrifuged at 250 x g for 5 minutes and the resultant supernatant aspirated. The pellet was resuspended in 20 ml of Growth Medium (DMEM-low glucose, 15 percent (v/v) fetal bovine serum [FBS; Hyclone, Logan, UT], 0.001% (v/v) 2-mercaptoethanol [Sigma], 1 ml per 100 ml of antibiotic [10,000 Units per ml penicillin, 10,000 micrograms per ml streptomycin]). The suspension was then passed through a 70 micron cell filter (BD, Franklin Lakes, NJ). Media volume was brought up to 50 ml with growth medium and then re-centrifuged at 250 x *g* for 5 min. The supernatant was aspirated and the cell pellet resuspended in growth medium. This process was repeated once more. Upon the final centrifugation, the supernatant was aspirated and the cell pellet was resuspended in 5 ml of fresh Growth Medium. The number of viable cells was determined following trypan blue staining using an improved Neubauer hemocytometer.

Cells were seeded initially at 3,000 or 5,000 cells per cm² on tissue culture treated flasks (Corning Inc., Coming, NY) previously coated with 2% (w/v) gelatin (Gelita porcine Type A: 250 Bloom, Sigma) for a minimum of 20 minutes and washed with PBS and grown at 37° C, 5% CO₂. Upon reaching 70-90% confluence, cells were passaged.

*Culture of porcine umbilicus-derived cells:* Following initial seeding, passaged cells (passage 1 and later) were routinely seeded at 5,000 cells/cm² onto T-75 or T-225 flasks (Coming Inc.) and cultured in a humidified incubator in atmospheric oxygen, 5% CO₂ at 37°C. Donors DD1, DD3, and DD4 were taken forward for further expansion.

Porcine umbilicus-derived cells (pUTCs) were first passaged when large colonies had formed or the flask was greater than 70 % confluent and in subsequent cultures every 3-4 days. Cells were passaged by aspirating off the medium, washing the cells with PBS and addition of 0.05 % (w/v) trypsin-EDTA (Invitrogen) to the cells. Following detachment of the cells, trypsin activity was inhibited by addition of Growth Medium. The cell suspension was removed from the flask and centrifuged in a sterile tube at 250 x *g* for 5 minutes. Following centrifugation, the supernatant was removed and pelleted cells were resuspended in Growth Medium. Cells were then counted using either trypan blue exclusion as before, or using Vi-Cell XR Cell Counting Apparatus (Beckman Coulter).

Donors DD1, DD3, and DD4 were expanded continuously from master bank (DD1, DD3) or from preclinical bank (DD4) to determine the time of senescence of pig UTC. In addition, we wanted to determine the growth rates of these cells as compared to previously expanded (hUTCs). The growth rates of pUTCs and hUTCs are shown in Figure 1. Morphologically, pUTCs appeared fibroblastic (Figure 2). An analysis of cell size, as determined by Vi-cell XR (Beckman Coulter), showed that all pUTC populations (DD1, DD3, and DD4) all had bimodal distributions similar to human umbilicus-derived cells (hUTCs) (Figure 3). Mean size analysis indicated that pUTC populations were in the range of 16 to 19 microns, whereas hUTCs fell in the range of 17 to 19 microns, further indicating the general similarity of these two populations.

*Immunophenotyping of pUTCs:* A number of antibodies were used to investigate the cell surface receptor expression and intracellular expression of proteins on pUTCs. This analysis was performed through multiple approaches: flow cytometry, immunocytochemistry, and tissue immunohistochemistry. Antibodies used are summarized in Tables 1 and 2. The list of markers used could be broken down into three main groups: (1) pig-specific markers to identify pUTC cultures; (2) human or rat-specific markers that are cross reactive for pig proteins to identify pUTC cultures; (3) human or rat-specific markers that do not cross react with pig proteins and therefore provide quality assurance that these cultures do not contain human or rat cell contaminants.

Flow cytometry: Analysis of cell surface receptors was performed in two ways. Cryopreserved pUTC were either thawed and immediately processed for flow cytometry, or they were plated on T-225 tissue culture flasks pretreated with gelatin for a period of 2-4 days at which time they were trypsinized and processed for flow cytometric analysis. Briefly, frozen aliquots of pUTCs of donor lots DD1, DD3, and DD4 were thawed by adding cryovials to a 37° C water bath for 2 minutes or until fully thawed at which time vials were transferred to a Biosafety cabinet for aseptic processing. Thawed cells were removed, the contents raised in Growth Medium, and then centrifuged at 250 x g for 5 minutes to remove residual DMSO. Upon completion of this process, cells were processed for flow cytometry as further described or they were plated at 5,000 cells / cm² and analyzed 2-4 days later following trypsinization and centrifugation.

Pelleted cells were re-suspended in 3% (v/v) FBS in PBS, and segregated into multiple flow tubes (BD Falcon). Five microliters antibody is next added to 100 microliters of cell suspension (1:20), and the cells were incubated in the dark for 30 min. at 4° C. After incubation, cells were washed with PBS and centrifuged at 250 x *g* for 5 min. to remove unbound antibody. Cells were resuspended in 500 microliters of PBS or PBS containing 3% (v/v) FBS and analyzed by flow cytometry using a FACScalibur instrument (Becton Dickinson, Franklin Lakes, NJ). In general, results of stained samples were compared to no primary controls (isotype appropriate) to establish differences between positive staining and background fluorescence levels.

Histograms of hUTCs at passage 14 analyzed by flow cytometry show positive expression of CD10, CD13, CD44, CD73, CD 90, and HLA-A, B, C, as noted by the increased detection of fluorescence relative to the IgG control (Figure 4). These cells were negative for CD31, CD34, CD45, CD117, and HLA-DR, DP, DQ as noted by fluorescence values consistent with the IgG control. Porcine UTCs also express CD90 and SLA Class I (HLA-A,B,C in humans), while lacking expression of CD31, CD45 and SLA Class II (HLA-DR,DP,DQ in humans). This was consistently observed for donors DD1, DD3, and DD4 (DD3 P8, Figure 5). We further analyzed pUTC banks for human- and rat-specific markers to ensure that they were not contaminated with cells derived from human or rat donors worked on in other areas of the laboratory. Negative staining ensured that pUTC banks were devoid of cross contaminating cells.

Immunocytochemistry: Briefly, frozen aliquots of pUTCs from working banks (20 +/- 4 PDL) of donor lots DD1, DD3, and DD4 were thawed by adding cryovials to a 37° C water bath for 2 minutes or until fully thawed at which time vials were transferred to a Biosafety cabinet for aseptic processing. Cells were plated on tissue culture flasks as previously described for one passage and the transferred to a 24 well tissue culture treated plate (Corning) pre-coated with gelatin. 48 to72 hours later, plates were fixed with cold 4% (w/v) paraformaldehyde (Sigma) for 10 minutes at room temperature. Immunocytochemistry was performed using antibodies directed against those antibodies described in Table 2.

Briefly, cultures were washed with PBS and exposed to a protein blocking solution containing PBS, 4% (v/v) goat serum (Invitrogen), and 0.3% (v/v) Triton (Triton X-100, Sigma) for 30 minutes at access intracellular antigens. Primary antibodies, diluted in blocking solution, were then applied to the cultures for a period of 1 hour at room temperature. Next, primary antibody solutions were removed and cultures washed with PBS prior to application of secondary antibody solutions (1 hour at room temperature) containing block along with the appropriate isotype specific antibody; in these cases, goat anti-mouse IgG - Texas Red or Alexa 488 (1:250, Molecular Probes, Eugene, OR), goat anti-mouse IgG1 - Texas Red or Alexa 488 (1:250 Molecular Probes, Eugene, OR), and/or goat anti-rabbit IgG - Texas Red or Alexa 488 (1:250, Molecular Probes). Cultures were then washed with PBS and 10 µM DAPI (Molecular Probes) applied for 10 minutes to visualize cell nuclei.

Following immunostaining, fluorescence was visualized using the appropriate fluorescence filter on a Nikon inverted epi-fluorescent microscope (Nikon, Lake Placid, NY). In all cases, positive staining represented fluorescence signal above control staining where the entire procedure outlined above was followed with the exception of application of a primary antibody solution (no 1° control). Representative images were captured using a digital color videocamera and ImagePro software (Media Cybernetics, Carlsbad, CA). For double and triple-stained samples, each image was taken using only one emission filter at a time. Layered montages were then prepared using ImagePro software or Adobe Photoshop software (Adobe, San Jose, CA).

Immunocytochemistry results confirmed high levels of CD90 expression, and little to no staining (< 2%) for CD31 and CD45 as previously observed using flow cytometry. An examination of intracellular markers showed high levels of vimentin and smooth muscle actin (SMA), but not cytokeratin 18 (CK18) expression. A pig-specific endothelial antibody was also used to assess the presence of endothelial cells, but results suggest that pUTC were negative for this marker (Figure 6). Together, pUTC expressed a variety of markers indicative of stromal cells, while lacking those of hematopoetic, endothelial, or epithelial origin, traits common to their human counterparts (Figure 7).

### Example 2

### Porcine umbilicus-derived cells are comparable to human umbilicus-derived cells

In this study the efficacy of pUTC was compared to hUTC in a 3-month RCS rat study. At postnatal Day 21 (P21) to P23, pigmented dystrophic RCS rats (P+) received single-dose subretinal injections ofpUTC in suspension (2 x 10⁴ cells/eye in 2 µL DMEM low-glucose medium) via a trans-scleral injection, as described in Lawrence *et al,* 2000. Untreated and vehicle-injected (DMEM low-glucose medium only) cohorts were included as controls (n=4). pUTC were thawed directly from cryopreservation and injected into the subretinal space (n=8). All RCS rats received daily dexamethasone injections (1.6 mg/kg, intraperitoneal) for 2 weeks and were maintained on cyclosporine A (210 mg/L) administered in the drinking water from 1 day prior to cell injection until euthanized. Optomotor threshold recordings were measured at and P100. The results were compared to results obtained from previous experiments with hUTC (Figure 8).

*Optomotor threshold recording:* A rotating cylinder covered with a vertical sine wave grating was calculated and drawn in virtual 3-dimensional space on 4 computer monitors arranged in a square. Rats standing unrestrained on a platform in the center of the square tracked the grating with reflexive head movements. Spatial frequency of the grating was clamped at the viewing position by repeatedly recentering the virtual "cylinder" on the head of the test subject. Head tracking (optomotor threshold - cycles/degree [c/d]) was quantified by increasing the spatial frequency of the grating until optomotor response could not be elicited. The grating optomotor threshold of rats was tested between 2 and 6 months of age. Normal animals demonstrate an optomotor threshold of 0.5 c/d at 3 months of age. A recording of 0.1 c/d is considered as functionally blind using this test. Subretinal hUTC injections (2 x 10⁴ cells) (0.4581 ± 0.01402 N=18) were compared with vehicle- controls (0.2147 ± 0.01663 N=22) and pig UTC (2x10⁴ cells) (0.4688 ± 0.02897 N=8). There was no significant difference in the optomotor test between the hUTC and the pUTC and both were significantly different from the controls (p<0.0001 for both). Data represented as mean ± SEM, unpaired t-test, comparison of hUTC and pUTC injected versus controls. These data suggest that the pig UTC are at least as efficacious in the optomotor test when compared to human UTCs.

### Example 3

### Rat umbilicus-derived cells are comparable to human umbilicus-derived cells

In an effort to isolate allogeneic equivalents to human umbilicus-derived cells (hUTCs), rat umbilical cord tissues from strain BD-IX were harvested, digested, expanded, and characterized. In order to examine the comparability of rat umbilicus tissue-derived cells (rUTC) to previously isolated hUTC, a series of tests was performed on these growing rat cultures that included (1) the monitoring of long-term growth, viability and cell morphology, (2) immunophenotyping of cell surface epitopes by flow cytometry and cytoskeletal markers by immunocytochemistry, and (3) PCR analysis of selected genes.

*Derivation of umbilicus-derived cells (UTC) from rat strain BD-IX:* Rat-derived UTC were isolated similarly to human derived UTC. Briefly, rat umbilical cords from BD-IX strains (Charles River Laboratories, Worcester, MA) were obtained via cesarean section approximately 2-6 days prior to full-term. Following cesarean section, cords were immediately transferred into sterile Growth Medium (DMEM-low glucose [Invitrogen, Carlsbad, CA], 15 percent (v/v) fetal bovine serum [FBS; Hyclone, Logan, UT], 0.001% (v/v) 2-mercaptoethanol [Sigma, St. Louis, MO], 1 mL per 100 mL of antibiotic [10,000 Units per mL penicillin, 10,000 micrograms per mL streptomycin]). Seven BD-IX cords were harvested and designated a unique identifier based on strain (BDIX), date of harvest (MMDDYY) following by a number (i.e. #1, 2, etc.) or a letter (i.e. A, B, or C).

The following process was repeated for each cord. The cord was washed in phosphate-buffered saline (PBS; Invitrogen) to remove blood and debris. The tissue was then mechanically dissociated in 500 cm² tissue culture plates using scalpels until the tissue was minced into a fine pulp. Chopped tissue was then transferred to 50 mL conical tubes containing a mixture of the enzymes collagenase (10 U/mL, Sigma, St. Louis, MO), dispase (12.6 U/mL, Roche Diagnostics, Indianapolis, IN), and hyaluronidase (1 U/mL, Vitrase®, ISTA Pharmaceuticals, Irvine, CA) diluted in Growth Medium. Tubes were incubated at 37° C in an orbital shaker (Barnstead Lab Line, Melrose Park, IL) at 225 rpm for 2 hours.

After digestion, enzymes were diluted with Growth Medium, and the mixture passed through a 100 µm filter (Becton Dickinson, Franklin Lakes, NJ). Tissue was then centrifuged at 250 x g for 5 minutes and the resultant supernatant aspirated. The pellet was resuspended in 10 mL of Growth Medium and this centrifugation/washing process repeated. Upon the final centrifugation, the supernatant was aspirated and the cell pellet resuspended in fresh Growth Medium. The number of viable cells was determined following trypan blue staining using an improved Neubauer hemocytometer.

Cells were seeded initially at 3,000 or 5,000 cells per cm² on tissue culture treated flasks (Coming Inc., Coming, NY) previously coated with 2% (w/v) gelatin (Gelita porcine Type A: 250 Bloom, Sigma) for a minimum of 20 minutes and washed with PBS and grown at 37° C, 5% CO₂. All cultures were grown in Growth Medium defined previously. Upon reaching 70-90% confluence, cells were passaged regularly on a 3-4 day schedule.

*Culture of umbilicus-derived cells (UTC) from rat strain BD-IX:* Following initial seeding, passaged cells (passage 1 and later) were routinely seeded at 5,000 cells/cm² onto T-75 cm² or T-225 cm² flasks (Corning Inc.) and cultured in a humidified incubator in atmospheric oxygen, 5% CO₂ at 37°C. In general, donor growth was variable with some donors exhibiting very poor growth. These donors were banked and or discarded, while donors exhibiting growth that allowed for regular passage were taken forward for later testing.

Rat umbilicus-derived cells were first passaged when large colonies had formed or the flask was >70 % confluent and in subsequent cultures every 3-4 days. Cells were passaged by aspirating off the medium, washing the cells with PBS and addition of 0.05 % (w/v) trypsin-EDTA (Invitrogen) to the cells. Following detachment of the cells, trypsin activity was inhibited by addition of Growth Medium. The cell suspension was removed from the flask and centrifuged in a sterile tube at 250 x g for 5 minutes. Following centrifugation, the supernatant was removed and pelleted cells were resuspended in Growth Medium. Cells were then counted using either a hemocytometer as before, or using Vi-Cell XR Cell Counting Apparatus (Beckman Coulter). This apparatus uses trypan blue exclusion in an automated fashion providing abbreviated reports of percentage viable, total viable cell number, and histogram plots of cell size distribution.

*Cryopreservation of umbilicus-derived cells (UTC) from rat strain BD-IX:* Rat umbilicus-derived cells were cryopreserved in cryomedium containing Growth Medium (90%, v/v) and DMSO (10%, v/v) (Sigma). Briefly, cells were trypsinized as normal, pelleted, counted, and then resuspended in cryomedium to generate vials of 0.5, 1, 2, 3, or 5 million cells. Initially, cells were immediately transferred to Mr Frosty (Nalgene) devices and placed in the -80° C freezer (Nalgene) to control the rate of freezing. Subsequently, a programmable freezer (Planer, model# 250-30, Middlesex, UK) was used. After the process of freezing was complete, frozen vials were transferred to a liquid nitrogen storage device maintained at -180° C.

In general, banks were generated (cryopreserved) at early (P1-4) and intermediate (P8-10) passage. Early banks were referred to as master banks and intermediate passage banks were referred to as preclinical or working banks. Working banks were, in general, 20 +/- 4 population doublings (PDL).

*Long-term Expansion of umbilicus-derived cells (UTC) from rat strain BD-IX:* To determine the growth and expansion potential of rUTC, cultures were passaged every 3-4 days following first passage and counted to determine resultant cell number. Each time, cells were reseeded at 5,000 cells/cm² in gelatin-coated flasks as before. In addition, cell morphology was investigated through visual inspection on a Nikon inverted phase contrast microscope (Nikon, Lake Placid, NY).

*Immunophenotyping of umbilicus-derived cells (UTC) from rat strain BD-IX:* A number of antibodies were used to investigate the cell surface receptor expression and intracellular expression of proteins in rUTC. This analysis was performed through multiple approaches: flow cytometry and immunocytochemistry. Antibodies used are summarized in Table 3.

Flow cytometry: Analysis of cell surface receptors was performed in two ways. Cryopreserved rUTC were either thawed and immediately processed for flow cytometry, or they were plated on T-225 tissue culture flasks pretreated with gelatin for a period of 2-4 days at which time they were trypsinized and processed for flow cytometric analysis.

Briefly, frozen aliquots of rUTCs from working banks (20 +/- 4 PDL) of donor lots BD-IX 033105 were thawed by adding cryovials to a 37° C water bath for 2 minutes or until fully thawed at which time vials were transferred to a Biosafety cabinet for aseptic processing. Thawed cells were removed, the contents raised in Growth Medium, and then centrifuged at 250 x *g* for 5 minutes to remove residual DMSO. Upon completion of this process, cells were processed for flow cytometry as further described or they were plated at 5,000 cells / cm² and analyzed 2-4 days later following trypsinization and centrifugation.

Pelleted cells were re-suspended in 3% (v/v) FBS in PBS, and segregated into multiple flow tubes (BD Falcon). Five microliters antibody was next added to 100 microliters of cell suspension (1:20), and the cells were incubated in the dark for 30 min. at 4° C. After incubation, cells were washed with PBS and centrifuged at 250 x *g* for 5 min. to remove unbound antibody. Cells were resuspended in 500 microliters of PBS or PBS containing 3% (v/v) FBS and analyzed by flow cytometry using a FACScalibur instrument (Becton Dickinson, Franklin Lakes, NJ). In general, results of stained samples were compared to no primary controls (isotype appropriate) to establish differences between positive staining and background fluorescence levels.

Immunocytochemistry: Analysis of intracellular proteins was performed through immunocytochemistry. In addition, cell surface receptor expression results from flow cytometry were confirmed using this approach.

Briefly, frozen aliquots of rUTCs from working banks (20 +/- 4 PDL) of donor lot BD-IX 033105 were thawed by adding cryovials to a 37° C water bath for 2 minutes or until fully thawed at which time vials were transferred to a Biosafety cabinet for aseptic processing. Upon cell processing as previously described, cells were plated on tissue culture flasks as previously described for one passage and then transferred to a 24 well tissue culture treated plate (Corning) pre-coated with gelatin. 48-72 hours later, plates were fixed with cold 4% (w/v) paraformaldehyde (Sigma) for 10 minutes at room temperature. Immunocytochemistry was performed using antibodies directed against those epitopes described in Table 3.

Briefly, cultures were washed with PBS and exposed to a protein blocking solution containing PBS, 4% (v/v) goat serum (Invitrogen), and 0.3% (v/v) Triton (Triton X-100, Sigma) for 30 minutes at access intracellular antigens. Primary antibodies, diluted in blocking solution, were then applied to the cultures for a period of 1 hour at room temperature. Next, primary antibody solutions were removed and cultures washed with PBS prior to application of secondary antibody solutions (1 hour at room temperature) containing block along with the appropriate isotype specific antibody (1:250, Molecular Probes, Eugene, OR). Cultures were then washed with PBS and 10 µM DAPI (Molecular Probes) applied for 10 minutes to visualize cell nuclei.

Following immunostaining, fluorescence was visualized using the appropriate fluorescence filter on a Nikon inverted epi-fluorescent microscope (Nikon, Lake Placid, NY). In all cases, positive staining represented fluorescence signal above control staining where the entire procedure outlined above was followed with the exception of application of a primary antibody solution (no 1° control). Representative images were captured using a digital color videocamera and ImagePro software (Media Cybernetics, Carlsbad, CA). For double and triple-stained samples, each image was taken using only one emission filter at a time. Layered montages were then prepared using ImagePro software or Adobe Photoshop software (Adobe, San Jose, CA).

*Results:* Flow cytometry analysis revealed that rat BD-IX UTC populations were positive for CD90 (97.5%), but few cells expressed markers of an endothelial phenotype (16.5%) (Figure 10). This is very similar to hUTC populations, whereby hUTC express CD90, but lack CD31. Immunocytochemical analysis further revealed that, similar to hUTC, rat UTC express smooth muscle actin and vimentin, but lack expression of cytokeratin 18. Immunocytochemistry was also used to confirm flow cytometry results of CD marker expression (Figure 11).

*PCR screening of umbilicus-derived cells (UTC) from rat strain BD-IX:* Total RNA isolation: One million cells were lysed by addition of 350 µL RLT Buffer (RNeasy Mini kit QIAGEN Cat# 74104). The lysate was applied onto a QIAshredder spin column placed in a 2 ml collection tube, and centrifuge for 2 min at maximum speed (18,000 x *g*; Microfuge 18 Centrifuge, Beckman-Coulter Cat# 367160). One volume of 70% (v/v) ethanol 200 proof (Sigma, Cat # E7023-500ML) was added to the homogenized lysate and applied to an RNeasy mini column placed in a 2 mL collection tube (supplied). The column was centrifuged for 15 seconds at ≥ 8000 x *g* (≥10,000 rpm). The flow through was discarded. Buffer RW1 (700 µl) was added to the RNeasy column and centrifuged for 15 seconds at ≥8000 x *g* (≥ 10,000 rpm) to wash the column. The flow through was discarded. The RNeasy column was transferred into a new 2 mL collection tube (supplied) and 500 µl Buffer RPE was applied to the RNeasy column. The column was centrifuged for 15 seconds at ≥ 8000 x *g* (≥ 10,000 rpm). The flow through was discarded. Another 500 µl of Buffer RPE was applied to the RNeasy column and centrifuge for 2 min at ≥ 8000 x *g* (≥ 10,000 rpm) to dry the RNeasy column. The RNeasy column was transferred to a new 1.8 mL collection tube (DNA LoBind tube 1.5 mL 22 43 102-1, Eppendorf AG) and centrifuged in a microcentrifuge at full speed for 1 min. To elute the RNA from the RNeasy column the column was transferred to a new 1.5 mL collection tube (supplied) and 30 µl RNase-free water applied before centrifugation for 1 min at ≥ 8000 x *g* (≥ 10,000 rpm) to elute. A second volume of RNase-free water was applied and centrifuged as before to elute into the same collection tube.

RNA quality and quantification: The quantity and quality of RNA was determined using Experion RNA StdSens Analysis Kit (Bio-Rad Cat # 700-7104; manufacturer instruction manual). Briefly, samples and standards were heated at 70°C for 2 minutes. Gel stain was applied to the standard sensitivity RNA chip in the Experion priming station (Bio-Rad Cat # 700-7030) and vortexed using the Experion Vortex station (Bio-Rad Cat #700-7040). Loading buffer, 5 µL was added to each well plus 1 µL sample RNA or the standard ladder was added to the appropriate wells and primed. The chip was loaded and run on the Experion automated electrophoresis station (Bio-Rad Cat # 700-7010) using the RNA StdSens analysis protocol.

First strand cDNA synthesis: The Superscript III First-Strand Synthesis system for RT-PCR kit (Cat# 18080-051, Invitrogen, Carlsbad, CA) was used to perform first strand cDNA synthesis. Briefly, the following reagents were combined in a 0.2 mL or 0.5 mL tube:
5 µg total RNA
50 µM oligo(dT)20, 2 µl
10 mM dNTP mix 2 µl
DEPC-treated water to 20 µl

The tube was then incubated at 65°C for 5 min. in a Thermal Cycler (Bio-Rad Cat #170-9703), then The 10x Thermal Ace Buffer kit place on ice for at least 1 min. The cDNA Synthesis Mix,
10X RT buffer 4 µl.
25 mM MgCl₂ 8 µl
0.1 M DTT 4 µl
RNaseOUT. (40 U/µl) 2 µl
Superscript. III RT (200 U/µl) 2 µl

The solution was then heated to 50°C for 50 min, 85°C for 5 min and placed on ice. 1 µl of RNase H was added each tube and incubated for 20 min at 37°C. The cDNA synthesis reaction was stored at -40°C.

Quantitative PCR: Quantitative PCR was performed on cDNA samples using Assays-on-Demand™ gene expression products: reticulon (Rn01648611_ml) and GAPDH (Rn99999916_s1; Applied Biosystems, Foster City, CA). Quantitative PCR was performed on ABI 7900HT Fast instrument. Initial denaturation was at 94°C for 20 seconds. Amplification was 94°C for 2 seconds, 60°C for 20 seconds for 40 cycles. The relative quantification was analyzed using GAPDH as calibrator and human fibroblast sample as control.

*Results:* The relative expression of reticulon was examined in a number of cell types and normalized to control human fibroblasts (Figure 12). Results indicate that there was a log increase in rat UTC reticulon expression relative to human fibroblasts and a two log increase in human UTC.

*Conclusion:* Compared with previously described human UTC, rat UTC maintain a similar morphology, grow at similar rates, and share a similar profile of expressed extracellular and intracellular proteins by flow cytometry and immunocytochemistry respectively. Furthermore, analysis of gene expression suggests that both rat and human UTC express reticulon, a signature gene in UTC, but previously shown to be lacking in human dermal fibroblasts, human mesenchymal stem cells, or human placental-derived cells. Together, these results suggest the comparability of rat UTC to previously characterized human UTC.

### Example 4

### Species specific upregulation of MHC Class II by IFN-γ

*IFN*-γ *activation of UTC:* Cells were thawed from liquid nitrogen and cultured in Hayflick growth media, consisting of Dulbecco's modified enriched media (Invitrogen), 15% fetal bovine serum (Hyclone), 1% penicillin/streptomycin (Invitrogen), and 0.001% β-mercaptoethanol (Sigma) for 3-5 days until 80% confluency. IFN-γ (Biosource International PSC4030) at 80ng/ml final concentration was added 72, 48 and 12 hour prior to harvest.

*MHC class II expression assessment on UTC:* Cells were harvested at these time points using TriplE Select (Invitrogen). The cells were adjusted to 1x10⁵/ml and incubated with anti SLA-DQ (BD Pharmingen) and anti SLA-DR (BD Pharmingen) with a rat anti-mouse IgG_{2a+b} phycoerythrin-conjugated secondary antibody (BD Pharmingen) and incubated for 30 minutes. After incubation, the cells were washed and kept at 4°C until analysis. A flow cytometer (FACScan) was setup for one color analysis and 30,000 cells were acquired. Data was plotted in histograms overlaying sample data and unstained cell data (Figure 13).

The data shows that IFN-γ is species specific in that rat or pig IFN-γ cannot upregulate MHC class II on human UTC. Since MHC class II plays an important role in the adaptive immune response it implies that xenogeneic models may underestimate the immunogenicity of UTC under inflammatory conditions. Furthermore, seemingly non immunogenic allogeneic cells may become immunogenic under inflammatory conditions.

### Example 5

### Method to assess allogeneic T cell responses to human umbilicus-derived cells in vitro

*Preparation of Peripheral Blood Mononuclear cells, T cells, and Antigen Presenting Cells:* Blood was collected from normal donors in heparinized tubes. Samples were diluted 2-5 times in PBS (phosphate buffered saline) without Mg²⁺ and Ca²⁺ (Invitrogen, 20012-027) and transferred into 50ml centrifuge tubes in 40ml aliquots. 10ml lymphocyte separation medium (MP Biomedical, 50494-36427) was underlayed for each tube. The tubes were centrifuged at 650 x *g* for 20 minutes without brake. White blood cells collected at the interface were carefully removed by pipette and collected in a fresh 50 ml tube. Identical samples were pooled and washed with PBS and centrifuged at 500 x *g* for 7 minutes. Cells were resuspended in PBS. A portion of the PBMC were reserved for counting and carboxyfluorescein diacetate succinimidyl ester (CFSE) staining. The remaining cells were divided up for T cell selection and antigen presenting cell (APC) isolation.

APC isolation was performed via plastic adherence. The PBMC intended for APC isolation were resuspended in warm Hayflick growth media, consisting of Dulbecco's modified enriched media (Invitrogen, 11885-084), 15% heat inactivated FBS (Hyclone, SH30070.03), 1% penicillin/streptomycin (Invitrogen, 15070-063), and 0.001% β-mercaptoethanol (Sigma, M7522) at 4x10⁶/ml and seeded on a pre-warmed T-75 culture flask (Coming, 430641.) The cells were incubated for 1-2 hours at 37°C, 5.0% CO₂. The non-adherent cells were then removed and reserved for T cell selection. The tissue culture flask was washed twice with warm PBS with Ca²⁺, Mg²⁺ (CellGro, 21-031-CM) followed by a wash with cold PBS without Ca²⁺, Mg²⁺. Washes were pooled with non-adherent cell portion. 10ml cold PBS without Ca²⁺, Mg²⁺ was added to the flask. The flask was incubated at 4°C for 15-20 minutes. Following the incubation the PBS was removed and transferred to a 50ml centrifuge tube. The flask was gently scraped with a cell scraper and washed twice with cold PBS without Ca²⁺, Mg²⁺. The suspension was centrifuged at 500xg for 7 minutes and resuspended in PBS for counting.

T cell isolation is a negative selection performed using the Miltenyi Pan T cell Isolation Kit II (Miltenyi Biotec, 130-091-156). The supernatant reserved from the APC isolation was centrifuged at 500xg for 7 minutes and resuspended at 40x10⁶/ml in Pan T cell Selection Buffer and incubated with 10µl Biotin-Antibody cocktail, provided by the kit, per 10x10⁶ PBMC for 10 minutes at 4°C. Afterwards 30µl of buffer per 10xl0⁶ PBMC was added to the suspension along with 20µl Anti-biotin microbeads per 10x10⁶ PBMC, also supplied by the kit. The suspension was incubated for 15 minutes at 4°C and then transferred to a 15ml centrifuge tube. 10-20X labeling volume of buffer was added to wash the cells, which were then centrifuged at 300xg for 10 minutes. The cells were then resuspended in 500µl buffer and run through the MACS LS column and MACS Separator. The column was washed three times with 1 ml buffer. The eluted T cells were then washed again with buffer and centrifuged at 500 x *g* for 7 minutes and resuspended in PBS for counting and CFSE-staining.

PBMC and T cells intended as responders were resuspended at 10x10⁶/ml in PBS and labeled with 2µl per 10⁶ cells of 5mM CFSE (Molecular Probes, C1157) diluted 1:1 with PBS for 7 minutes. Labeling was stopped by adding 1ml FBS per 50x10⁶ cells and incubating for 1 minute. Labeled cells were washed with PBS and centrifuged at 500 x *g* for 7 minutes. The PBMC and T cells that would not be seeded with APC were resuspended at 1x10⁶/ml in growth media, while T cells, intended to be cultured with APC, were resuspended at 2x10⁶/ml and the APC, not labeled with CFSE, were resuspended at 1x10⁶/ml. The cells were either cultured in Hayflick growth media (MSCGM), (MSC growth media: Cambrex, PT-3001), or AIM-V serum free lymphocyte media (Invitrogen, 12055-091).

*Preparation and activation of MSC and UTC:* MSC (Cambrex lot: 4F0591) or UTC (lot: 25126057) obtained from thaw or passage were seeded at 5,000 per cm² on 48-well plates (Costar, 3548) coated with 2% porkskin gelatin (Gelita USA Inc.) in either Hayflick media or MSCGM. Cells were incubated at 37°C, 5.0% CO₂ in a humidified incubator. After 5 days, recombinant human IFN-γ (Pierce, RIFNG50) was added at 500U/ml. Cells were cultured for an additional 48 hours prior to co-culture. Cells were then washed with PBS and trypsinized using TrypLE Select (Invitrogen, 12604-013). Once the cells detached from the wells they were washed with media and centrifuged at 250xg for 5 minutes and resuspended in FACSFlow Sheath fluid (BD Pharmingen, 342003.) Cells were stained with FITC (fluorescein isothiocynate)-conjugated anti-HLA-DR,DP,DQ (BD Pharmingen, 555558) for 30 minutes at 4°C then washed with sheath fluid and centrifuged at 250 x *g* for 5 minutes. Cells were brought to a final volume of 300 µl sheath fluid and acquired on a flow cytometer (BD FACSCaliber) to detect MHC class II expression.

*Preparation of co-cultures and addition of anti-CD28 mAb:* Co-cultures consisted of 1x10⁶ CFSE-labeled responders cultured with confluent nonirradiated IFN-γ-activated or unactivated MSC or UTC for a total volume of 1ml per well in gelatin-coated 48-well plates in Hayflick media, MSCGM, or AIM-V media. Cultures that contained T cells with APC consisted of 0.5ml of T cells from the 2x10⁶/ml suspension and 0.5ml of the APC suspension for a total volume of 1 ml. Purified anti-CD28 mouse anti-human monoclonal antibody was added to designated T cell cultures at 1µg/ml. All cultures set up in duplicate. Plates were incubated at 37°C, 5.0% CO₂ in a humidified incubator for 5 days.

*³H-Thymidine incorporation:* After 0.5ml of each culture was removed for cytokine analysis, the wells were harvested and trypsinized to remove all the cells and resuspended in the original volume of 1ml media. Triplicate volumes of 100 µl for each culture were transferred to a 96 well clear flat bottom plate (Costar.) ³H-thymidine (Perkin Elmer, NET-027) was added to each well at a concentration of 1µCi/ml. The plates were incubated at 37°C, 5.0% CO₂ for 6 hours. The cultures were then run through 96 well filter plates (Perkin Elmer, 6005174) using a microplate scintillation reader (Packard, TopCount NXT.)

*CFSE Flow cytometry:* After samples were taken for ³H-thymidine incorporation, the cultures were centrifuged at 500xg for 7 minutes and resuspended in 100 µl FACS buffer, consisting of FACSFlow sheath fluid and heat inactivated 3% FBS. The cells were aliquoted into FACS tubes and stained with the following monoclonal antibodies (BD Pharmingen):

| | |
|---|---|
| Anti-CD4 PE | Anti-CD25 PE |
| Anti-CD3 PE-Cy5 | Anti-CD45RA PE-Cy5 |
| Anti-CD8 APC | Anti-CD45RO APC |

Cells were incubated for 30 minutes with the antibodies at 4°C then washed with FACS buffer and centrifuged at 500 x *g* for 7 minutes. Cells were brought to a final volume of 300 µl FACS buffer and 30,000 events per sample were acquired on the flow cytometer.

Recombinant human IFN-γ was tested at concentrations of 500U/ml and 50U/ml on the MSC for 24h, 48h, and 72h in MSCGM (data not shown). Although MHC class II expression could be seen with IFN-γ concentration at 50U/ml, optimal concentration and activation period was found to be 500U/ml for 48 hours. MHC class II expression of the UTC, which were cultured in Hayflick, is comparable to that of the MSC (Figure 14). Notably, MHC I was upregulated too after IFN-γ activation (data not shown in this example).

³H-thymidine incorporation showed MSC induce a vigorous response in PBMC but not with purified T cells when cultured in MSCGM, and to a lesser extent, the Hayflick media. PBMC seeded alone also significantly proliferated, most likely due to the rich MSCGM, causing high background in the analysis. Despite the background, the effects of addition of anti-CD28 antibody to the T cells could be observed. Addition of anti-CD28 antibody restored proliferative activity of T cells, as shown in Figure 15. When the cells were cultured in serum free AIM-V media, minimal proliferation of PBMC was observed compared to that of cultures in MSCGM.

CFSE-flow cytometry confirmed the data shown above of PBMC proliferation when seeded on MSC in MSCGM. Again, T cells alone showed minimal proliferation; however, upon addition of purified anti-CD28 antibody, some proliferation was restored, with more dividing cells being non-CD4+, most likely CD8⁺ (Figure 16).

In general, experiments conducted using AIM-V media resulted in lower proliferation observed in both ³H-thymidine incorporation analysis and CFSE flow cytometry. Based on CFSE flow cytometry, proliferative activity was restored upon addition of APC to purified T cells; however, proliferation appears to occur earlier than that of PBMC (Figure 17).

The results indicate that hUTC and MSC can induce T cell proliferation in vitro independent of antigen processing but dependent on costimulatory signaling. This was further enhanced when MHC class II was upregulated. Therefore it is hypothesized that UTC and MSC require APC and inflammatory cytokines present that can upregulate MHC class II (e.g. IFN-γ) in order to induce an adaptive immune response. Therefore cells injected in a non-inflammatory environment and an environment lacking costimulatory signals may protect hUTC from immune recognition by the adaptive immune system.

### Example 6

### Immunogenicity assessment of porcine umbilicus-derived cells in vitro

*Preparation of PBMC:* Umbilical cord derived tissue cells have been isolated from inbred miniswine. This model was used to study whether UTC could induce an immune response when injected into a fully mismatched allogeneic recipient. In addition, an assay was developed to assess immunogenicity of UTC *in vitro.* PBMC isolated from UTC-injected animals were co-cultured with the UTC and cell proliferation was quantified via CFSE flow cytometry and ³H-thymidine incorporation to measure an immune response. Furthermore MHC differences between host and donor play an important role in transplantation rejection, MHC class II is an especially important driver of the allogeneic immune response. UTC do not intrinsically express MHC class II molecule on their surface, but will express the molecule when exposed to the inflammatory cytokine IFN-γ. In this study cell proliferation in response to allogeneic stimulation and the role of IFN-γ therein was investigated. The experimental animals were either CC or AC haplotype injected subcutaneously with DD PBMC or pig UTC according to Table 4.

Blood was drawn from naïve CC and AC animals as controls. Blood was taken from DD pigs to serve as a stimulator in the experiments. Blood from an outbred Yucatan (Yuc) pig was also taken as a third party control. Samples were diluted 2-5 times in phosphate buffered saline (PBS) without Mg²⁺ and Ca²⁺ (Invitrogen, 20012-027) and transferred into 50ml centrifuge tubes in 40ml aliquots. 10ml lymphocyte separation medium (MP Biomedical) was underlayed for each tube. The tubes were centrifuged at 650 x *g* for 20 minutes without brake. White blood cells collected at the interface were carefully removed by pipette and collected in a fresh 50 ml tube. Identical samples were pooled and washed with PBS and centrifuged at 500 x *g* for 7 minutes. Cells were resuspended in 5 ml ACK lysing buffer (Cambrex, 10-548E) at room temperature. After 5 minutes, the cells were centrifuged at 500xg for 7 minutes and resuspended in PBS. PBMC intended as stimulators were irradiated at 2500 rad.

PBMC intended as responders were resuspended at 10e6/ml in PBS and labeled with 2µl per 10⁶ cells of 5mM CFSE diluted 1:1 with PBS for 7 minutes. Labeling was stopped by adding 1ml heat-inactivated fetal bovine serum (FBS) per 50x10⁶ cells and incubating for 1 minute. Labeled cells were washed with PBS and centrifuged at 500 x *g* for 7 minutes. Both responders and stimulators were resuspended at 2x10⁶/ml in Hayflick growth media, consisting of Dulbecco's modified enriched media (Invitrogen, 11885-084), 15% FBS (Hyclone, SH30070.03), 1% penicillin/streptomycin (Invitrogen, 15070-063), and 0.001% β-mercaptoethanol (Sigma, M7522).

*Preparation of UTC:* UTC from thaw or passage were seeded at 5,000 per cm² on 48-well plates (Costar, #3548) coated with 2% porkskin gelatin (Gelita USA Inc.) in Hayflick growth media. Cells were incubated at 37°C, 5.0% CO₂. After 5 days, recombinant swine IFN-γ (Biosource International, PSC4030) was added at 80ng/ml. Cells were cultured for an additional 48 hours and then used for co-culture experiments. A few wells per assay were harvested in order to verify MHC class II upregulation. The wells were washed with PBS and trypsinized using TrypLE Select (Invitrogen). Once the cells detached from the wells they were washed with media and centrifuged at 250xg for 5 minutes and resuspended in FACSFlow Sheath fluid (BD Pharmingen, 342003.) Cells were stained with anti-SLA-DQ (BD Pharmingen, 551538) and anti-SLA-DR (BD Pharmingen, 553642) with a rat anti-mouse IgG_{2a+b} phycoerythrin-conjugated secondary antibody (BD Pharmingen, 340269) for 30 minutes at 4°C then washed with sheath fluid and centrifuged at 250xg for 5 minutes. Cells were brought to a final volume of 300 µl sheath fluid and acquired on a flow cytometer (BD FACSCaliber) to detect MHC class II expression.

*Mixed Lymphocyte reaction (MLR) and co-cultures:* MLR consisted of 1x10⁶ CFSE-labeled responders (cells from experimental CC/AC animals) cultured with 1x10⁶ irradiated stimulators (naïve CC/AC, DD or Yuc) for a total volume of 1ml per well in uncoated 48-well plates. 1x10⁶ CFSE-labeled responders were also cultured with confluent wells of UTC (activated with IFN-γ as well as unactivated control.) The UTC were not irradiated. The volume was brought up to 1ml with Hayflick growth media in the co-cultures with UTC. All cultures were set up in duplicate and incubated at 37°C, 5.0% CO₂ in a humidified incubator for 5 days. MHC class II expression and MHC I upregulation was observed in cells treated with 40ng/ml recombinant IFN-γ. Optimal concentration and activation period was found to be 80ng/ml for 48 hours (Figure 18).

*³H-Thymidine incorporation:* Triplicate volumes of 100 µl of cell suspension from each culture were added to a 96 well clear flat bottom plate (Costar, 3595.) Since the proliferating cells tightly adhere to UTC the wells were harvested and trypsinized to remove all the cells and resuspended in the original volume of 1ml media. ³H-thymidine (Perkin Elmer, NET-027) was added to each well at a concentration of 1µCi/ml. The plates were incubated at 37°C, 5.0% CO₂ for 6 hours. The cultures were then run through 96 well filter plates (Perkin Elmer, 6005174) using a microplate scintillation reader (Packard, TopCount NXT.). In general, ³H-thymidine incorporation shows that PBMC isolated from CC/AC animals that received DD PBMC have a higher response against DD PBMC and both activated and unactivated UTC. There also seems to be an overall higher response against the UTC than the PBMC (Figure 19).

*CFSE Flow Cytometry:* After samples were taken for ³H-thymidine incorporation, the cultures were centrifuged at 500 rcf for 7 minutes and resuspended in 100 µl FACS buffer, consisting of FACSFlow sheath fluid and 3% heat-inactivated FBS. The cells were aliquoted into FACS tubes and stained with anti-CD4a PE (BD Pharmingen, 559586) and biotinylated anti-CD8a (BD Pharmingen, 551305) with APC (allophycocyanin)-conjugated streptavidin (BD Pharmingen, 554067.) Cells were incubated for 30 minutes with the antibodies at 4°C then washed with FACS buffer and centrifuged at 500 x g for 7 minutes. Cells were brought to a final volume of 300 µl FACS buffer and 30,000 events per sample were acquired on a flow cytometer.

CFSE-flow cytometry showed high cell proliferation for cells cultured with PBMC or activated UTC expressing MHC class II. For all co-cultures, activated UTC induced a higher immune response than that of unactivated UTC (Figure 20).

A significant portion of the proliferating cells is CD8+ (Figure 22). However, this population is present across all samples, particularly with the naive, self, UTC, and the third party, implying that this is an aspecific response.

Results showed that pig PBMC components do proliferate in response to stimulation by allogeneic pUTC. The results show that both CD4+ and CD8+ T cells proliferated in response to PBMC and pUTC stimulation in vitro. This proliferation was generally enhanced when MHC class II was upregulated on UTC. These results are similar to the results seen with the human UTC and the immune response is most likely induced via similar mechanism.

### Example 7

### Detection of immune responses elicited by allogeneic porcine umbilicus-derived cells in vivo

*³H Mixed Lymphocyte Reaction (MLR):* For one-way mixed leukocyte reaction (MLR) response cultures, responder peripheral blood mononuclear cells (PBMC), drawn from animals that had been injected with pUTC previously, were plated in triplicate in 96-well flat-bottom plates at a final concentration of 4 × 10⁵ cells/well and were stimulated by an equal number of irradiated (25 Gy) stimulator PBMC. The medium consisted of RPMI 1640 supplemented with 6% fetal pig serum, 10 mM HEPES, 1 mM glutamine, 1 mM sodium pyruvate, 0.1 mM nonessential amino acids, 100 U/mL penicillin, 100 µg/mL streptomycin, 50 µg/mL gentamicin, and 2x10⁵ M 2-mercaptoethanol. These MLR co-cultures were incubated for 2 and 5 days at 37°C in 6% CO₂ and 100% humidity. 3H-thymidine was added for the last 6 hours of culture and wells were harvested onto Mash II glass fiber filters and counted for beta emission and expressed in counts per minute.

The MLR can be used to assess whether *in vivo* sensitization by pUTC has occurred. T cells present in blood samples from sensitized animal have an earlier response in vitro due to immunological memory (Memory T cells) and show a higher response in the day 2 MLR than non- sensitized animals. The day 2 / day5 response ratio can be used to assess whether an animals has been sensitized.

### Example 8

### Method to detect immune responses elicited by allogeneic porcine umbilicus-derived cells in vivo by measuring the serum antibody response

Antibody response to porcine umbilicus-derived cells was assayed by flow cytometry using sera collected at serial time points from injected animals to stain PBMC that were haplotype matched to UTC (SLA^{dd}).

Briefly, 10 µl of serum from each recipient was added to 1x10⁶ cells of SLA^{dd} PBMC. Following 30 minutes incubation, cells were washed twice prior to incubation with fluorescein conjugated secondary antibodies. Sera from previously immunized animals were used as a positive control. Detection of antibody was reported as a difference in mean fluorescence intensity when compared to the pre-treatment sample. Antibody response to porcine umbilicus-derived cells was also confirmed by testing sera with known reactivity to "DD" PBMC on "DD" porcine umbilicus-derived cells. 10 µl of serum was incubated with 1x10⁵ porcine umbilicus-derived cells for 30 minutes followed by another 30-minute incubation with the fluorescein conjugated secondary antibodies. Again, detection of antibody was reported as a difference in mean fluorescence intensity when compared to the pre-treatment sample.

This method is capable of detecting serum antibodies directed against pig or human UTC (Figure 22).

### Example 9

### Method to detect immune responses elicited by allogeneic porcine umbilicus-derived cells in vivo by measuring the cytotoxic T cell response

*T cell Mediated Cytotoxicity Assay via RT-CES:* This system measures the presence of adherent cells (i.e. UTC) to the bottom of a 96-well microelectronic sensing plate (E-plate). Cytotoxic T cells contained within PBMC samples can potentially kill UTC when immunological memory has developed after *in vivo* administration of UTC. If UTC cells are killed by the cytotoxic T cells the readout signal will change. To assess whether pigs that were injected with UTC developed cytotoxic T cell responses directed against UTC, PBMC were harvested from blood samples and co-cultured with UTC grown to confluence on E-plates.

Porcine umbilicus-derived cells from thaw were seeded at 5,000 per cm² in growth media on a 96-well microelectronic sensing plate (E-plate) with cell index readings taken every hour by the RT-CES. After 2-3 days, recombinant swine IFN-γ was added to select wells containing UTC and cultured for an additional 48 hours prior to the start of the co-culture in order to also test the influence of IFN-γ in this test system.

In order to be able to assess the cytotoxic T cells response these cells have to be restimulated in vitro with cells from the same origin as the UTC before they can be co-culture with the UTC grown on the E-plates. Therefore, PBMC obtained from experimental animal blood samples were co-cultured with irradiated (25 Gy) stimulator "DD" PBMC ("DD" is the origin of the UTC which were derived from "DD" umbilical cords). After 7 days these PBMC co-cultures were harvested and the isolated cells were transferred to the E-plate containing UTC at a final concentration of 2 × 10⁵ or 1 × 10⁵ cells/well with readings taken every 30 minutes by the RT-CES to assess cytotoxic kill.

*Results:* The Cell Index (CI) is the real-time measurement of cell-attaching, spreading, morphological dynamics, and correlated with cell number changes. CI is an arbitrary unit translated from the electronic readout: CI = (Zcell - Zmedium)/Zmedium, Z representing impedance. Percent kill of the UTC was calculated from the change in CI due to kill of UTC 4 hours after the addition of effector cells: % Kill = (CI0 - CI4h)/CIO (Figure 23).

*Conclusion:* This method can measure kill of UTC by cytotoxic T cells avoiding radioactive chromium labeling as is done classically. We showed that animals sensitized to UTC animals do have circulating cytotoxic T cells circulating in their blood that are capable to kill UTC.

### Example 10

### Method to detect immune responses elicited by allogeneic porcine umbilicus-derived cells in vivo by measuring the cytotoxic antibody response

Detection of cytotoxic antibodies to cell surface antigens was performed using an antibody/complement reaction, followed by a dye exclusion assay and FACS acquisition.

Target cells, obtained from processed PBMC from SLA^{dd} animals, were diluted to a concentration of 5 x 10⁶ cells/ml concentration in Medium 199, supplemented with 2% fetal calf serum (FCS). Serum samples are serially diluted in a 96-well round bottom plate and incubated with 25 µl of the target cell suspension, for a total volume of 50 µl per well. A negative control of media alone and a positive control consisting of serum from a previously immunized animal are used for each assay. Following 15 minutes of incubation at 37°C, each well is washed with 125µl of media and then centrifuged at 1200rpm for 5 minutes at 4°C. Diluted rabbit complement is added to each well, and plates are then incubated at 37°C for 30 minutes. Cells are then stained with 7-AAD, and then acquired by flow cytometry. A gate is drawn on a FSC versus SSC plot to include all the targets and exclude any debris (Figure 24, panel a). This gated population is then plotted on a FSC versus FL3 and gated on FL3 positive to determine the percent of apoptotic cells (Figure 24, panel b) in the entire population. The percent of apoptotic cells was then plotted against the dilution factor.

This method demonstrated the presence of cytotoxic antibodies in serum from animals sensitized with pUTC (Figure 24, panel c). The serum antibodies were toxic for cells of the same origin as the UTC ("DD") and in particular to cells that expressed "DD" MHC class I molecules. This indicates that the cytotoxic serum antibodies in this donor/recipient combination were mainly directed against the MHC class I molecule.

### Example 11

### Method to detect sensitization of the recipient by allogeneic porcine umbilicus-derived cells using a skin transplant rejection assay

Animals were tested for immunocompetence and for sensitization from the pUTC injection using a pUTC haplotype-matched SLA^{dd} skin graft.

*Method:* Split thickness skin grafts are obtained from both a donor animal and the experimental animal itself using a dermatome. Skin is then placed on a graft bed, also prepared with the dermatome on the dorsum of the recipient animal. Grafts are monitored daily to determine acceptance or rejection of the skin based on three characteristics: texture, color, and temperature.

Typical results observed are shown in Figure 25. Grafted skin from "DD" origin was acutely rejected when grafted on UTC sensitized animals whereas this skin was rejected at a normal tempo when grafted onto non-sensitized animals. This demonstrated the utility of the skin grafting procedure to assess the immune status of animals, injected with UTC, *in vivo.*

### Example 12

### Adverse immune responses elicited by systemic and subcutaneous administration of allogeneic porcine umbilicus-derived cells

Porcine umbilicus-derived cells were harvested fresh from culture and infused into recipient pigs at a total dose of 1x10⁸ cells for both subcutaneous or intravenous administration. Cells were resuspended in Lactated Ringer's solution at a concentration of 1x10⁷ cells/ml. For IV injections (n=2), porcine umbilicus-derived cells were slowly infused at a concentration of 1 x 10⁷ cells /ml in a total volume of 10cc through the IV catheter. The cells were then rinsed through the catheter with an additional 3cc Lactated Ringer's solution. For SC injections, porcine umbilicus-derived cells (n=2) and IFN-γ activated porcine umbilicus-derived cells (n=2) were also resuspended in Lactated Ringer's solution at a concentration of 1 x 10⁷ cells/ml in a total volume of 10cc. Cells were activated with IFN-γ by seeding the porcine umbilicus-derived cells at a density of 5,000 per cm² in growth media and incubated at 37°C, 5.0% CO₂ for 5 days. Cells were then treated with 25ng/ml and recombinant swine IFN-γ for 48 hours.

A total of four skin injection sites were used per animal. Sites were injected subcutaneously with 2.5cc of the cell suspension using a 25 gauge needle. The experimental procedure is outlined in Table 5 and Figure 26.

*Investigation of the Allogeneic Pig Anti pig UTC Immune Response:* In these studies, the immunogenicity of porcine umbilicus-derived cells was investigated in a well-studied, fully allogeneic mismatched, large animal model using inbred mini swine. The porcine umbilicus-derived cells were obtained from a "DD" haplotype pig, and cultured to obtain sufficient numbers. Recipient pigs of "CC" haplotype received a fully allogeneic "DD" injection of 100 x 10⁶ porcine umbilicus-derived cells intravenously, subcutaneously, subretinally or subcutaneously multiple times. Blood samples were taken at predetermined intervals and the immune response of the porcine umbilicus-derived cell recipients against donor cells was assessed via mixed lymphocyte cultures, cytotoxic T cell assays and serum antibody assays as described *supra.* Furthermore, a matched and a fully mismatched (allogeneic) skin graft from same donor strain as the porcine umbilicus-derived cells was placed on the recipients to confirm *in vitro* results (Table 6).

*Results:* The following gives a summary of the in vivo study findings per group. The results are summarized as follows: serum antibody assays (Figure 27), cytotoxic antibody detection (Figure 28), and correlation between serum antibodies and skin graft rejection (Table 6)

*IV infusion of pUTC (n=2):* When 100x10⁶ pUTC where injected IV no increased or accelerated T cell proliferation was detected in the MLR. Furthermore, there were no Ab against the donor haplotype ("DD") detected in the serum samples. A fully allogeneic "DD" skin graft was rejected at a normal tempo.

*SC injection of UTC (n=2):* In this experiment 100x10⁶ UTC were injected SC. No increased or accelerated T cell response was detected and serum samples did not contain detectable levels of anti "DD" antibodies. The "DD" skin graft was rejected at a normal tempo.

*SC injection ofPB, MC (n=2):* In this experiment 100x10⁶ "DD" PBMC were injected SC as a control. An increased and accelerated T cell response was detected in the MLR. The serum samples contained detectable levels of anti "DD" antibodies which were complement fixing. The animals did not receive a skin graft.

*SC injection in inflammatory environment (n=2):* In this experiment 100x10⁶ "DD" UTC were injected SC in an inflammatory environment. An increased and accelerated T cell response was detected in the MLR. The serum samples contained detectable levels of anti "DD" antibodies which were complement fixing. The pigs rejected the "DD" skin graft in an accelerated tempo.

*SC injection of UTC exposed to IFN*-γ *prior to injection (n=2):* UTC were exposed in culture to IFN-γ 48 hours prior to injection and 100x10⁶ of these activated "DD" UTC were injected SC. An increased and accelerated T cell response was detected in the MLR and the T cells were cytotoxic. The serum samples contained detectable levels of anti "DD" antibodies which were complement fixing. The pigs rejected the "DD" skin graft in an accelerated tempo.

*Investigation of the specificity of the Immune response:* Finally, serum samples containing anti "DD" antibodies and peripheral blood samples from animals sensitized to "DD" were further analyzed using recombinant animals that either shared the "D" haplotype for either the Class I MHC ("DC") or the Class II MHC ("CD"). The antibody binding assay show significant binding to cells with the D haplotype for class I and class II, minimal binding to cells that share the MHC II haplotype and no binding by the control serum obtained at the time of skin graft. Therefore the serum antibodies are mainly induced by the MHC I differences between the "DD" and "CC" molecules in this strain combination. (Figure 29 and Table 7). In addition, the complement fixing serum antibody tests (Figure 30) and cytotoxic T cell assay (Figure 31) show that the Class I of "D" MHC molecules induces a T cell response that is cytotoxic and that anti "DD" serum antibodies causes preferential lysis of targets expressing "D" of class I and not "D" of class II.

*Conclusion: In vivo* studies showed no immune response for IV or SC injections. However, an adaptive immune response is evident for SC with complete Freund's adjuvant (CFA), SC with IFN-γ treated UTC. Our in vivo data demonstrated that a single injection of allogeneic pUTC injected either IV or SC did not lead to a detectable adaptive immune response. The IV injection experiment showed what would happen if all the injected cells would end up in the bloodstream. Finally, we demonstrated that IFN-γ, present in inflammatory environments, alters the immunogenicity of the UTC. In order to avoid an immune response against the injected UTC injecting into such environments should be avoided or proper immunomodulatory agents should be administered.

### Example 13

### Multiple injections of allogeneic porcine umbilicus-derived cells

*Method:* Pigs (n=2) received repeated SC injections as described previously. A total of three doses of cells (100x10⁶ UTC per injection) were administered separated at one-month intervals. The first two doses were injected into the same site, while the third dose was injected at a disparate site.

*Results:* After one injection, no increased or accelerated or cytotoxic T cell response was detected and serum samples did not contain detectable levels of anti "DD" antibodies similar to the previous single injection ofpUTC S.C. A second injection of 100x10⁶ UTC was given 1 month later SC in the same area as the first injection. After injection an increased and accelerated T cell response was detected in the MLR and the T cells were cytotoxic. The serum samples contained detectable levels of anti "DD" antibodies which were complement fixing. Finally, a third injection of 100x10⁶ UTC was given SC far removed from the first two injections. The increased and accelerated T cell response remained detectable in the MLR and the T cells were cytotoxic. The serum samples contained detectable levels of anti "DD" antibodies which were complement fixing. Although the response the second injection was not resolved at the time of the third injection the immune response after the third injection appeared similar level or higher than after the second injection. The "DD" skin grafts were rejected in an accelerated tempo and the grafts had a whitish appearance.

Data of all previous examples involving in vivo pig UTC studies are summarized in Table 8.

*Conclusion:* This *in vivo* study showed that an adaptive immune response against allogeneic pUTC can occur when pUTC are administered multiple times.

### Example 14

### Ocular administration of allogeneic porcine umbilicus-derived cells

The objective of this study was to evaluate the potential toxicity of human and pig-derived umbilicus cells (hUTC, referred to as CNTO 2476) and (pUTC), respectively) when injected into the intravitreal or subretinal space of the Göttingen Minipig.

The minipig is an accepted non-rodent species for use in ocular toxicology studies. The minipig also permits monitoring of the umbilicus cell in an allogeneic setting by using the pig cell which is analogous to the human umbilicus cell, as well as evaluation of the human umbilicus cell in a xenogeneic setting. The protocol for administration into the animals is summarized in Table 9.

Dosing of the animals was carried out over a 7-day period. Topical ophthalmic antibiotic (Gentamicin) was applied to the right eye only for Groups 1-7, or both eyes for Group 8, once on the day before treatment. Gentamicin was also applied to the treated eye immediately following the injection and twice on the day following the injection (AM and PM). The right eye received the control (Group 8) or test article (Groups 1-7) and the left eye remained untreated (Groups 1-7) or received control article (Group 8). An analgesic, (buprenorphine, 0.01 mg/kg) was administered by intramuscular injection to each animal following completion of the procedure and again once the following day. Animals were fasted overnight prior to the administration of control or test article and associated anesthesia procedures outlined below.

*Subretinal injection procedure:* Prior to dosing, mydriatic drops (1% Mydriacyl and 2.5% phenylepherine) were applied to the treated eye. Prior to dosing, the animals received an intramuscular injection of a sedative cocktail of ketamine (22 mg/kg), glycopyrrolate (0.01 mg/kg) and acepromazine (1.1 mg/kg). An isoflurane/oxygen mix was administered to the animals via an endotracheal tube during the procedure. For the treated eye, the conjunctivae was flushed with benzalkonium chloride (Zephiran^{™}) diluted in Sterile Water, U.S.P. to 1:10,000 (v/v) then a topical anesthetic (Proparacaine, 0.5%) was applied. Each animal was positioned under an operating microscope. A 180° conjunctival peritomy was performed exposing the superonasal and temporal quadrants. An inferotemporal sclerotomy was made, and using a 25G cannulation system, an infusion port/cannula was inserted transconjunctivally at approximately 2-5 mm from the limbus. Two other cannulas were inserted in a similar fashion and positioned for the light pipe and vitrector. Balanced salt solution (BSS)+ was used for infusion. A standard 3-port core vitrectomy was done.

For the cell injection, one of the cannulas was removed, the conjunctiva was opened and the sclerotomy opening extended to allow insertion of the 30G cannula A bent 30-gauge metal cannula was used to create a retinotomy and a small (approximately 20 µL) bleb of PBS was created. Immediately after creation of the small bleb, the test or control article was injected into the bleb.

*Anti-pig or Human UTC IgG or IgM Determination:* IgM and IgG antibodies specific for pig (Group 3 and 8) and human (Group 5) umbilical-derived tissue cells (hUTC) have undergone a preliminary evaluation. IgM and IgG antibodies specific for pig (Group 1, 2, 4, and 8) and human (Group 5 and 7) umbilical-derived tissue cells (hUTC) were assessed in all animals from the respective groups. Blood samples (approximately 2.5 mL) were collected in SST tubes via the jugular vein prior to the start of the dosing, on Days 7, 14, 28 and at the end of the observation period of each group (3 or 6 months). Immediately after collection, each blood sample was placed on wet ice and transferred to the immunology laboratory. Following blood clotting, serum was separated from whole blood by centrifugation (1200g for 10 minutes at approximately 4°C), aliquoted and stored at approximately -80°C until analysis.

Anti-pig pUTC or anti-human hUTC IgM and IgG antibody levels were determined separately using validated flow cytometry methods. Cell cultures of pig or human UTC cells were stimulated or not with Interferon gamma (if required in order to induce an increase in the level of MHC class II) 48 hours prior to the staining.

*Results:* The presence of anti-UTC IgM antibodies in 3/6 control animals demonstrated a significant false positive result for that parameter. Only the Group 6 and Group 7 (CNTO 2476) animals had a greater incidence of 5/6 and 4/6 positive results, respectively. Care must therefore be taken in the interpretation of the IgM results.

For IgG, Group 6 animals 601 and 605 and Group 7 animal 705 were the only CNTO 2476 animals to have noticeable levels above the baseline. No animals were found to markedly differ from pre-dose with regards to anti-pUTC IgG antibody levels. (See Figure 32 and 33).

In summary, the most immunologically reactive groups appeared to be Groups 6 and 7 (hUTC) that exhibited both IgM and IgG antibody response. Only two Group 6 and one Group 7 animal exhibited anti-CNTO 2476 IgG antibodies. No pUTC animals exhibited an IgG response. Although a relation to the pUTC or CNTO 2476 cannot be ruled out, the presence of IgM antibodies in 50% of the control animals renders the results of similar or lesser incidence in some of the pUTC groups (1.5 x 10⁶ cells subretinal or 3 x 10⁵ cells intravitreal) or the low dose CNTO 2476 group (3 x 10⁵ cells subretinal) to be equivocal.

*Conclusion:* No anti-donor serum antibodies where detected when the pigs had been injected s.r. with allogeneic pUTC as measured by flow cytometry. However, some pigs injected with human UTC s.r. developed detectable serum antibodies directed against human UTC. This finding emphasized the need for allogeneic test models in order to design proper clinical trials and avoid exposure of the patient to potentially unnecessary immunosuppressive drugs.

### Example 15

### Allogeneic and Xenogeneic antibody response in observed in SD rats

Sprague Daley rats were injected with BDIX rat UTC, BDIX rat PBMC, BDIX rat splenocytes, or hUTC without immunosuppression. Serum samples were taken from the animals every seven days for four weeks and tested for IgM and IgG against both activated and unactivated BDIX UTC to determine an allogeneic response. By Day 21 the rats injected with either PBMC or splenocytes showed both an IgM and an IgG response against only activated UTC expressing MHC class II. Serum from the group that received human UTC was tested against activated and unactivated human UTC where a response was detected against both as early as Day 7, indicating a xenogeneic response. The antibody reactivity against both activated and unactivated cells suggests that the immune response is against MHC class I, or both class I and class II, or a non-MHC molecule. Based on these results another xenogeneic study was conducted in which rats were injected with either human MSC or human UTC with or without CsA. Serum from rats that did not receive CsA treatment, injected with either MSC or UTC, showed a response against their respective injected cell type. This also shows that CsA is sufficient to block the antibody response in this xenogeneic model.

*Preparation of rat BDIX UTC, human MSC, human UTC:* Rat BDIX UTC, human UTC (lot: 25126078), and human MSC (Cambrex lot: 4F0591) from thaw or passage were seeded at 5,000 per cm² on 48-well plates (Costar, 3548) coated with 2% porkskin gelatin (Gelita USA Inc.) in Hayflick growth media. Cells were incubated at 37°C, 5.0% CO₂ in a humidified incubator. After 5 days, recombinant rat IFN-γ (Pierce, RR-2007-20) was added at 5000U/ml, 500U/ml, and 50U/ml to the rat UTC. The rat UTC were cultured for an additional 24, 48, and 72 hours. Recombinant human IFN-γ (Pierce, RIFNG50) was added at 500U/ml to the human MSC and human UTC the human cells which were then cultured for another 48 hours, this having been determined to be the optimal activation condition for human cells. All the cells were then washed with PBS and trypsinized using TrypLE Select (Invitrogen, 12604-013). Once the cells detached from the wells they were washed with media and centrifuged at 250 x g for 5 minutes and resuspended in FACSFlow Sheath fluid (BD Pharmingen, 342003.) Rat cells were stained with FITC (fluorescein isothiocyanate)-conjugated anti-RT1D (BD Pharmingen, 550982) and human cells were stained with FITC-conjugated anti-HLA-DR,DP,DQ (BD Pharmingen, 555558) for 30 minutes at 4°C then washed with sheath fluid and centrifuged at 250xg for 5 minutes. Cells were brought to a final volume of 300 µl sheath fluid and 10,000 events per sample were acquired on a flow cytometer (BD FACSCaliber) to detect MHC class II expression.

*Serum antibody FACS:* UTC were prepared as mentioned above but not stained for RT1D. Heat inactivated serum drawn from the experimental animals was heat inactivated for 30 minutes at 56°C. 10µl of each serum sample was mixed with activated and unactivated UTC in separate tubes. Serum incubated with cells for 30 minutes at 4°C and then washed with sheath fluid and heat inactivated 3% fetal bovine serum (Hyclone, SH30070.03). 2µl of anti-rat IgG₁₊₂ₐ FITC (BD Pharmingen, 553881) and 2µl of anti-rat IgM PE (BD Pharmingen, 553888) secondary antibodies were added to each tube and incubated for 30 minutes at 4°C and then washed with sheath fluid and heat inactivated 3% fetal bovine serum. Cells were brought to a final volume of 300 µl sheath fluid and acquired on a flow cytometer.

*Results: MHC Class II expression on BDIX rat UTC:* MHC class II expression was detected as early as 24 hours after activation with IFN-γ at a concentration as low as 50U/ml. Optimal concentration and activation period was found to be 50U/ml for 72 hours. However, in subsequent studies, cells were activated using IFN-γ at a concentration of 500U/ml for 48 hours (Figure 34).

*Presence of anti-BDIX rat UTC IgG and IgM:* Serum drawn at Day 7 and Day 14 were tested together with a secondary antibody control. Serum from rats that were injected with splenocytes tested positive for IgM against activated BDIX UTC with a complete peak shift and a slight shift for the IgG peak. There is a slight peak shift for both IgM and IgG against activated UTC in serum from rats that were injected with PBMC at Day 14. Serum drawn at Day 7 from any animal did not test positive for either immunoglobulin isotype. Serum drawn on both Day 7 and Day 14 from all rats tested negative against unactivated UTC for both IgM and IgG.

Serum drawn on Day 21 and Day 28 were tested separately with pre-treatment serum as a baseline control. At Day 21 the presence of both IgM and IgG was evident for rats injected with PBMC or splenocytes, but only against activated UTC (See Figures 35 and 36). Animals that were injected with UTC did not show antibody response to activated or unactivated UTC at any time point.

By Day 28, IgM and IgG concentrations were returning to basal levels for rats that were injected with splenocytes. Serum drawn from rats that were injected with PBMC, however, showed peak concentrations of both antibodies at Day 28. Again, no antibody response was observed when tested on unactivated UTC (Figures 37 and 38).

*MHC Class II expression on human MSC and human UTC:* MHC class II expression was induced on both the human MSC and human UTC by IFN-γ and verified before testing the serum drawn from rats in the xenogeneic study (Figure 39).

*Presence of anti-human MSC and UTC IgG:* Serum drawn from rats that were injected with human UTC were tested against human UTC lot 25126057. There was aspecific binding of the anti-rat IgM secondary antibody (data not shown) ⁽⁶⁾, however there was a shift as early as Day 7 and complete shifts in IgG for serum drawn on Days 14, 21, and 28 when tested on both activated and unactivated human UTC. Since the antibodies bind both activated and unactivated UTC it cannot be concluded whether the antibodies are directed against MHC class I, class II or another non-MHC molecule (Figure 40).

*Effect of CsA treatment on anti-human MSC and UTC antibody response:* Rats were injected with either human MSC or human UTC with or without CsA treatment in study P-2005-506. Serum drawn from these rats were tested on either human MSC or human UTC, both activated and unactivated. Serum was tested at Days 7 and 14 and compared to a pre-treatment baseline control. However, IgG was found in the serum drawn only from rats that did not receive CsA treatment for both MSC and UTC injected rats. There was a minimal shift, if any, seen in serum drawn on Day 7; however, the IgG peak shift was evident by Day 14 (Figure 41 and 42) and seemed stronger for MSC. Table 10 and Table 11 summarize the results.

*Conclusion:* The results showed that rUTC did not induce anti-donor antibodies where control, MHC class II expressing, rat PBMC and spleen cells did induce antibodies directed against IFN-γ activated, MHC class II expressing, UTC but not to unstimulated UTC. This emphasizes the importance of MHC class II as an antigen in the allogeneic response and that the lack of expression may protect the cells from an adaptive immune response.

The human UTC did induce anti donor serum antibodies in this xenogeneic model. However these antibodies recognized both IFN-γ activated and unactivated UTC and MSC. Therefore it is not clear whether these antibodies were directed to MHC discrepancies or other non-MHC cell antigens.

Finally, it was demonstrated that rats treated with Cyclosporine A did not show measurable anti xenogeneic donor UTC or MSC serum antibodies. This suggests that Cyclosporine A is sufficient in blocking a xenogeneic antibody response against UTC or MSC in this rat model.

### Example 16

### Additional immunogenicity studies with UTC in rat recipients

Several studies have been conducted using rats as recipients of human, pig or rat Umbilicus derived cells. In these studies serum samples were collected and analyzed for the presence of serum antibodies directed against the injected umbilicus derived cell as described in previous examples.

*Jugular injections:* Female nude rats with an implanted jugular cannula were obtained from Charles River Laboratories, in the 175-200 gram range, received a 0.5 ml jugular injection (5 million cells in PBS) at a rate of 0.5 ml/Min. Blood (target volume 250 µl) was drawn at day-1, 0, 7,14 and 28 via the retro-orbital sinus. Serum was isolated from the blood samples and stored at -80°C until analysis.

*Subcutaneous injection:* Normal SD rats were injected SC with 100µl cell suspension containing 0.5x10⁶ cells. Blood (target volume 250 µl) was drawn at day-1, 0, 7,14 and 28 via the retro-orbital sinus. Serum was isolated from the blood samples and stored at -80°C until analysis.

*Intracardiac injections:* Rats received a permanent left descending artery (LAD) ligation. Rats with a left ventricular ischemic visual grade (immediately post ligation) of 2 or less were negated from the study. A total of 1X10⁶ cells were injected into 2 sites (25µl each site) at the level of the papillary muscle bordering the ischemic area of the left ventricle 10 minutes post LAD ligation. Serum samples were collected from all rats at 7, 14, 21 and 28 days and stored at -80°C until analysis. Rat study results from previous examples are summarized in Table 10 and Table 11.

*Conclusion:* Xenogeneic cells induce and immune response when injected into the rat whereas allogeneic cells do not induce an immune response unless injected into an inflammatory area. The rat model mirrors the pig model with regard to the immune response and is a small animal alternative to the large pig model.

### Example 17

### Studies of the effect of timing and site of multiple subcutaneous injections on the adaptive immune response in a multi-dose large animal model

Previous experiments showed that a second injection of UTC subcutaneously, in the same location as the primary injection, induced an adaptive immune response. One hypothesis is that the first SC injection primes the injection site without inducing an adaptive immune response. A second injection into this now primed environment however leads to a primary adaptive immune response.

This study investigated the contribution of the timing of the subsequent injections, and the choice of the site of the subsequent injection on the adaptive immune response observed. 1) The timing between the injections was varied, and 2), UTCs were injected into different sites. The experimental groups were as follows:

GROUP 1 (n=4): Repeat SC injection with varied timing for re-injection: animals were re-injected at 3 months (n=2) or at 6 months (n=2) after the first dose of UTC.

GROUP 2 (n=2): Repeat SC injection into varied sites: animals were re-injected at 1 month after the first dose of UTC, but at a different and far removed from the first injection site (n=2).

GROUP 3: Repeat SC injection into the same SC area as the first injection but with immunomodulation: animals were re-injected at 1 month after the first dose of UTC, in the same SC location but an immunomodulatory treatment covering the second injection (high dose prednisone for 7 days with a 7 day taper) (n=2) or covering both the first and second injection with an immunomodulatory regiment (short course cyclosporine and prednisone).

*Multiple Injections ofp UTC S.C:* For each injection, pUTC were harvested fresh from culture and injected into recipient pigs at a total dose of 1x10⁸ cells for SC administration. Cells were resuspended in Lactated Ringer's solution at a concentration of 1x10⁷ cells/ml in a total volume of 10cc. A total of 4 skin injection sites were injected subcutaneously with 2.5cc of the cell suspension using a 25-gauge needle. Two doses of cells were administered separated at 3-month interval (n=2), 6-month interval (n=2), 1-month interval with predisone immunosuppression at the second injection (n=2), and 1-month interval with cyclosporine A (15-30mg/kg divided in two doses/day for 400-600ng/ml for 7 days with a 7 day taper) and predisolone (12.5mg/kg @ 2 doses/day for 7 days with a 7 day taper) administered via a gastric tube at both injections (n=2). Both doses were injected into the same site. Two doses of cells were administered at 1-month interval, the doses injected at separate sites (n=2). All these animals (Table 12) were monitored at predetermined time points for immune responses using our follow up procedure (Figure 43).

*Antibody Detection by Flow Cytometry:* Antibody response to UTC was assayed by flow cytometry using sera collected at serial time points from injected animals to stain PBMC that were haplotype matched to UTC (SLA^{dd}). Briefly, 10 µl of serum from each recipient was added to 1x10⁶ cells of SLA^{dd} PBMC. Following 30 minutes incubation, cells were washed twice prior to incubation with fluorescein conjugated secondary antibodies to swine IgG or IgM. Sera from previously immunized animals were used as a positive control. Detection of antibody was reported as a difference in mean fluorescence intensity when compared to the pre-treatment sample. Antibody response to UTC was also confirmed by testing sera with known reactivity to "DD" UTC. Briefly, UTC are cultured and activated with recombinant swine IFN-γ to upregulate MHC class I expression and induce MHC class II expression. 10 µl of serum was incubated with 1x10⁵ pUTC for 30 minutes followed by another 30-minute incubation with the fluorescein conjugated secondary antibodies. Again, detection of antibody was reported as a difference in mean fluorescence intensity when compared to the pre-treatment sample (Figure 44).

Antibodies against rUTC were measured using the same technique. Briefly, rUTC are cultured and activated with recombinant rat IFN-γ to upregulate MHC class I expression and induce MHC class II expression. Sera from experimental animals are incubated with 1x10⁵ rUTC followed by incubation with fluorescein- or phycoerythrin-conjugated secondary antibodies to rat IgG or IgM. Again, detection of antibody was reported as a difference in mean fluorescence intensity when compared to the pre-treatment sample.

*³H Mixed Lymphocyte Reaction (MLR):* For one-way mixed leukocyte reaction (MLR) response cultures, responder peripheral blood lymphocytes (PBL) were plated in triplicate in 96-well flat-bottom plates at a final concentration of 4 × 10⁵ cells/well and were stimulated by an equal number of irradiated (25 Gy) stimulator PBL. The medium consisted of RPMI 1640 supplemented with 6% fetal pig serum, 10 mM HEPES, 1 mM glutamine, 1 mM sodium pyruvate, 0.1 mM nonessential amino acids, 100 U/mL penicillin, 100 µg/mL streptomycin, 50 µg/mL gentamicin, and 2x10⁵ M 2-mercaptoethanol. Cultures were incubated for 2 and 5 days at 37°C in 6% CO2 and 100% humidity. ³H-thymidine was added for the last 6 hours of culture and wells were harvested onto Mash II glass fiber filters and counted for beta emission and expressed in counts per minute.

*Skin Grafting:* Animals were tested for immunocompetence and for sensitization from the pUTC injection using a pUTC haplotype-matched SLA^{dd} skin graft. Split thickness skin grafts are obtained from both a donor animal and the experimental animal itself using a dermatome. Skin is then placed on a graft bed, also prepared with the dermatome on the dorsum of the recipient animal. Grafts are monitored daily to determine acceptance or rejection of the skin based on three characteristics: texture, color, and temperature (Figure 45). Rejection was rated normal or accelerated based on rejection measured by naïve control animals, 17476 and 17506.

*Results:*

*3-month interval without immunosuppression (n=2):* Animals were injected subcutaneously with 100x10⁶ pUTC and received a second dose of 100x10⁶ pUTC in the same location 3 months later. No increased or accelerated T cell proliferation was detected in the MLR, however there were Ab against the donor haplotype ("DD") detected in the serum samples after the second injection (Figure 46). A fully allogeneic "DD" skin graft was rejected at an accelerated tempo.

*6-month interval without immunosuppression (n=2):* In this experiment animals again received two doses of 100x10⁶ UTC in the same location, subcutaneous, with a 6-month interval. No increased or accelerated T cell response was detected by MLR but, again, serum samples contained detectable levels of anti "DD" antibodies after the second injection (Figure 46). The "DD" skin graft was rejected at an accelerated tempo.

*1-month interval with prednisone at second injection (n=2):* In this experiment animals received two doses of 1 00x10⁶ UTC in the same location, subcutaneous, with a 1-month interval and prednisone administration at the second injection. No increased or accelerated T cell response was detected in the MLR but serum samples contained detectable levels of anti "DD" antibodies after the second injection (Figure 47). The "DD" skin graft was rejected at an accelerated tempo.

*1-month interval with prednisolone and cyclosporine A at both injections (n=2):* In this experiment animals received two doses of 100x10⁶ UTC in the same location, subcutaneous, with a 1-month interval and cyclosporine A and prednisone administration at both injections. No increased or accelerated T cell response was detected by MLR and serum samples did not contain detectable levels of anti "DD" antibodies after either injection (Figure 47). The "DD" skin graft was rejected at a normal tempo.

*1-month interval at separate sites (n=2):* Animals were injected subcutaneously with 100x10⁶ pUTC and received a second dose of 100x10⁶ pUTC in the different location 1 month later. No increased or accelerated T cell response was detected in the MLR but serum samples contained detectable levels of anti "DD" antibodies after the second injection. The "DD" skin graft was rejected at an accelerated tempo.

These results are summarized in Tables 13 and 14.

*Conclusion:* These in vivo results confirmed previous findings that the initial injection of allogeneic pUTC injected SC does not induce a detectable T cell or serum antibody response. However, subsequent doses showed a clear systemic immune response in this SC pig model that can be averted with immunosuppression.

### Example 18

### Studies on the effect of timing of multiple subretinal injections on the adaptive immune response in a multi-dose large animal model

Previous experiments showed that a second injection of UTC subcutaneously could induce an adaptive immune response. This study was designed to assess whether multiple injections in the eye will induce an adaptive immune response. The following parameters were tested: interval between first and second injection, varying sites of the second injection (optional) and whether prior sensitization appears to be a contraindication.
*Multiple Injections of pUTC S.R.:* Pigs were injected subretinally with 300,000 thawed pUTC using a previously described method (Example 14).

The following experimental groups were used (Table 15):

GROUP 1: PBMC were injected subretinally (SR) into sensitized animals and re-injected (SR) at 3 months (n=2). Animals were sensitized with 100x10⁶ PBMC injected subcutaneous four weeks prior to subretinal injections.

GROUP 2: UTC were re-injected SR at 3 months and 3rd SR injection at 6 months (n=3).

GROUP 3: UTC were re-injected SR at 6 months (n=3).

Peripheral blood and serum samples were collected from all animals and analyzed for serum antibodies against pUTC and Mixed Lymphocyte Responses, at predetermined time points (Figure 48). In addition all animals were observed and assessed frequently for inflammation or other potential negative impacts on the eye.

*Results:* PBMC administered SR after systemic sensitization (group 1) does show an increase in serum IgG (Figure 49) however there were no inflammatory responses detected in the treated eye other than responses attributed to the surgical procedure. Animals receiving multiple SR doses of pUTC either spaced 3 months apart (Figure 50) or 6 months apart (Figure 51) did not show elevated serum IgG or accelerated MLR responses (data not shown) against pUTC levels.

*Conclusion:* Our in vivo data confirmed previous findings that a single injection of allogeneic pUTC injected SR does not induce a detectable adaptive immune response. Further investigating the effect of subsequent doses in these animals shown in this report showed that multiple SR injections did not provoke an immune response. This, most likely, can be attributed to the eye's immune privileged environment.

Publications cited throughout this document are hereby incorporated by reference in their entirety. Although the various aspects of the invention have been illustrated above by reference to examples and preferred embodiments, it will be appreciated that the scope of the invention is defined not by the foregoing description but by the following claims properly construed under principles of patent law.

**Table 1 Antibodies used for flow cytometric analysis of pUTCs**

| **Antibody** | **Specificity** | **Isotype** | **Vendor (Catalog #)** |
|---|---|---|---|
| hCD13-PE | human | mIgG1 | BD Pharmingen (555394) |
| pCD 16-PE | pig | mIgG1 | Serotec (MCA1971 PE) |
| hCD31-PE | human | mIgG1 | BD Pharmingen (555446) |
| rCD31-PE | rat, pig | mIgG1 | BD Pharmingen (555027) |
| rCD44-PE | rat | mIgG1 | Serotec (MCA643PE) |
| p/r CD44 | pig | mIgG1 | Abcam (AB23844) |
| pCD45-FITC | pig | mIgG1 | Serotec (MCA1222F) |
| hCD90-PE | human, pig | IgG1 | BD Pharmingen (555596) |
| rCD90-PE | rat | mIgG1 | BD Pharmingen (554898) |
| SLA Class I | pig | mIgG1, | BD Pharmingen (552547) |
| SLA DQ | pig | mIgG2a | BD Pharmingen (551538) |
| SLA DR | pig | mIgG2a | BD Pharmingen (553642) |

| *Isotype Controls* | | | |
|---|---|---|---|
| mIgG1-PE | | | BD Pharmingen (550083) |
| mIgG2a-PE | | | Invitrogen (P21139) |
| mIgG1-FITC | | | Zymed (04-6111) |

**Table 2 Antibodies used for immunocytochemical and histological analysis**

| **Antibody** | **Specificity** | **Isotype** | **Vendor (Catalog #)** |
|---|---|---|---|
| pCD16-PE | pig | mIgG1, 1:40 | Serotec (MCA1971 PE) |
| rCD31-PE | rat, pig | mIgG1, 1:40 | BD Pharmingen (555027) |
| hCD34-PE | human | mIgG1, 1:100 | BD Pharmingen (550761) |
| rCD44-PE | rat | mIgG1, 1:40 | Serotec (MCA643PE) |
| p/r CD44 | pig | mIgG1, 1:40 | Abcam (AB23844) |
| pCD45-FITC | pig | mIgG1, 1:40 | Serotec (MCA1222F) |
| hCD90-PE | human, pig | mIgG1, 1:40 | BD Pharmingen (555596) |
| rCD90-PE | rat | mIgG1, 1:40 | BD Pharmingen (554898) |
| cytokeratin18 | human, rat, pig | mIgG1, 1:200 | Chemicon (MAB3234) |
| bFGF | broad | rbIgG, 1:250 | Chemicon (AB1458) |
| vimentin | human, rat, pig | mIgG1, 1:250 | Sigma (V6630) |
| desmin | human | rbIgG, 1:100 | Sigma (D8281) |
| smooth muscle actin | human, rat * | mIgG2a, 1:200 | Sigma (A2547) |
| pEndothelial | pig | IgG1, 1:100 | Serotec (MCA1222F) |
| vWF | human | rbIgG, 1:100 | Sigma (F3520) |

**Table 3 Antibodies used for flow cytometric & immunocytochemical analysis of rUTCs**

| **Antibody** | **Specificity** | **Isotype** | **Vendor (Catalog #)** |
|---|---|---|---|
| CD31-PE | rat | mIgG1 | BD Pharmingen (555027) |
| CD90-PE | rat | mIgG1 | BD Pharmingen (554898) |
| | | | |
| Cytokeratin 18 | human, rat | mIgG1, 1:200 | Chemicon (MAB3234) |
| Vimentin | human, rat | mIgG1, 1:250 | Sigma (V6630) |
| Smooth muscle Actin | human, rat * | mIgG2a, 1:200 | Sigma (A2547) |
| | | | |

| *Isotype Controls* / *No primary Controls* | | | |
|---|---|---|---|
| mIgG1-PE | | | BD Pharmingen (550083) |
| mIgG1-Alexa 488 | | | Molecular Probes (A21121) |
| mIgG2a-PE | | | Molecular Probes (P21139) |

| | | | |
|---|---|---|---|
| * not indicated on technical sheet provided from vendor but known to react from rat ICC results | | | |

**Table 4: Outline of the groups used in the immunogenicity study described in Example 6**

| Pig ID | Subcutaneous injection |
|---|---|
| 16650 | DD4 UTC |
| 16707 | DD PBMC |
| 16633 | DD3 UTC |
| 16792 | DD PBMC |

**Table 5: Summary of assays performed in Example 12**

| Animal | Cells | MLR | Serum Antibody | Cytotoxic Antibody | Cytotoxic T cells | Skin Graft |
|---|---|---|---|---|---|---|
| 16707 | PBMC 1 | X | X | X | Not done | Not done |
| 16792 | PBMC 2 | X | X | X | Not done | X |
| 16649 | UTC IV 1 | X | X | X | Not done | X |
| 16481 | UTC IV 1 | X | X | X | Not done | Not done |
| 16650 | UTC SC 1 | X | X | X | Not done | Not done |
| 16633 | UTC SC 2 | X | X | X | Not done | X |
| 16843 | CFA/UTC 1 | X | X | X | X | X |
| 16844 | CFA/UTC 2 | X | X | X | X | X |
| 16854 | aUTC 1 | X | X | X | X | X |
| 16922 | aUTC 2 | X | X | X | X | X |
| 17025 | UTC SCx3 | X | X | X | X | X |
| 17026 | UTC SCx3 | X | X | X | X | X |

**Table 6: Summary of skin graft results in relation to the presence of serum antibodies against DD. The presence of serum antibodies correlated with accelerated skin graft rejection. Serum antibodies were detected when UTC were injected into an inflammatory environment (16843; 16844; 16854 and 16922) or when injected multiple times (17025 and 17026) but not when injected SC only once (16649 and 16481).**

| Animal | UTC source | Antibody prior to skin graft | Clinical pattern of skin graft rejection (start to completion day) | Antibody after skin graft / type |
|---|---|---|---|---|
| 16649 | Unactivated UTC | No | Normal (6-8) | Yes - IgM and IgG |
| 16481 | Unactivated UTC | No | Normal (5-8) | Yes - IgM and IgG |
| 16843 | Unactivated UTC around CFA | Yes | Accelerated (4-6) | Yes - IgG |
| 16844 | Unactivated UTC around CFA | Yes | Accelerated (2-6) | Yes - IgG |
| 16854 | Activated UTC | Yes | Accelerated (2-6) | Yes - IgG |
| 16922 | Activated UTC | Yes | Accelerated (1-6) | Yes - IgG |
| 17025 | Unactivated UTC x3 | Yes | Accelerated (1-5) | Yes - IgG |
| 17026 | Unactivated UTC x3 | Yes | Accelerated (1-6) | Yes - IgG |

**Table 7 shows the binding of serum antibodies to PBMC from recombinant haplotype animals ("DD" full mismatch; CD class II mismatch and DC class I mismatch). In general, serum antibodies bound to cells that were mismatched for MHC class I indicating that this molecule is recognized as the foreign molecule in this combination (DD donor to CC recipient) and drives the antibody response.**

| Serum antibody (IgG) directed against SLA^{I} | | | | |
|---|---|---|---|---|
| | | DID^{II} | C^{I}D^{II} | D^{I}C^{II} |
| Animal ID | Timepoint | Mismatch for I and II | Mismatch for II | Mismatch for I |
| 16854 | Day 18 | + | - | - |
| Act. UTC | Day 0 skin graft | + | - | - |
| | Day 8 skin graft | ++ | + | ++ |
| 16922 | Day 18 | + | - | + |
| Act. UTC | Day 0 skin graft | + | - | + |
| | Day 8 skin graft | ++ | + | ++ |
| 16843 | Day 21 | + | - | + |
| CFA/UTC | Day 0 skin graft | + | - | - |
| | Day 8 skin graft | ++ | + | ++ |
| 16844 | Day 21 | + | - | + |
| CFA/UTC | Day 0 skin graft | + | - | - |
| | Day 8 skin graft | ++ | + | ++ |

**TABLE 7:** Serum antibody (IgG) directed against SLA^{I} Scoring based on Figure 26.

**Table 8: shows the summary of pig experiment assay results**

| Animal | Cells | Time point | D2 or D3 response to DD PBMC | D5 Response to DD PBMC | Antibody response post-injection | Cytotoxic antibody post-injection | Cytotoxic T cell response |
|---|---|---|---|---|---|---|---|
| 16707 | PBMC | Pre-treatment | No | Yes | Yes | Yes | N/A |
| | 1 | D30 | No | Yes | | | |
| 16792 | PBMC | Pre-treatment | N/A | N/A | Yes | Yes | N/A |
| | 2 | D30 | No | Yes | | | |
| 16649 | UTC | Pre-treatment | No | Yes | No | No | N/A |
| | IV 1 | D30 | No | Yes | | | |
| 16481 | UTC | Pre-treatment | No | Yes | No | No | N/A |
| | IV 1 | D30 | No | Yes | | | |
| 16650 | UTC | Pre-treatment | No | Yes | No | No | N/A |
| | SC 1 | D30 | No | Yes | | | |
| 16633 | UTC | Pre-treatment | No | Yes | No | No | N/A |
| | SC2 | D30 | No | Yes | | | |
| 16843 | CFA/ | Pre-treatment | No | Yes | Yes | Yes | Yes (after |
| | UTC 1 | D30 | No | Yes | | | skin graft) |
| | | Post skin graft | Yes | Yes | | | |
| 16844 | CFA/ | Pre-treatment | No | Yes | Yes | Yes | Yes (after |
| | UTC 2 | D30 | No | Yes | | | skin graft) |
| | | Post skin graft | Yes | Yes | | | |
| 16854 | aUTC | Pre-treatment | N/A | Yes | Yes | Yes | Yes (after |
| | 1 | D60 | Yes | Yes | | | D60) |
| | | Post skin graft | Yes | Yes | | | |
| 16922 | aUTC | Pre-treatment | N/A | Yes | Yes | Yes | Yes (after |
| | 2 | D60 | No | Yes | | | D60) |
| | | Post skin graft | Yes | Yes | | | |
| 17025 | UTC | Pre-treatment | No | Yes | Yes (after | Yes (after | Yes (after |
| | SC x3 | 1^{st} injection | No | Yes | 2^{nd} | 2^{nd} | 2^{nd} |
| | 1 | 2^{nd} injection | Yes | Yes | injection) | injection) | injection) |
| | | 3^{rd} injection | Yes | Yes | | | |
| | | Post skin graft | Yes | Yes | | | |
| 17026 | UTC | Pre-treatment | No | Yes | Yes (after | Yes (after | Yes (after |
| | Sc x3 | 1^{st} injection | No | Yes | 2^{nd} | 2^{nd} | 2^{nd} |
| | 2 | 2^{nd} injection | Yes | Yes | injection) | injection) | injection) |
| | | 3^{rd} injection | Yes | Yes | | | |
| | | Post skin graft | Yes | Yes | | | |

**Table 9: The dosing protocol for the experiments described in Example 14.**

| Groups-treated Eye (number of cells)^{a} | Total Number of Animals | Route of Administration | Dose Volume (µl/eye)^{b} | 3-month Observation Period? | 6-month Observation Period? |
|---|---|---|---|---|---|
| 1: pUTC (6x10⁴) | 6 | Subretinal | 100 | 3 | 3 |
| 2: pUTC (3x10⁵) | 6 | Subretinal | 100 | 3 | 3 |
| 3: pUTC (1.5x10⁶) | 6 | Subretinal | 100 | 3 | 3 |
| 4: pUTC (3x10⁵) | 6 | Intravitreal | 100 | 3 | 3 |
| 5: CNTO 2476 (3x10⁵) | 6 | Subretinal | 100 | 3 | 3 |
| 6: CNTO 2476 (1.5x10⁶) | 6 | Subretinal | 100 | 3^{d} | 3 |
| 7: CNTO 2476 (3x10⁵ ) | 6 | Intravitreal | 100 | 3^{d} | 3 |
| | | Subretinal/ | 100/ | | |
| 8: Vehicle (PBS)^{c} | 6 | Intravitreal | 100 | 3 | 3 |

| | | | | | |
|---|---|---|---|---|---|
| * cells injected into one eye and the contralateral eye remained untreated (except Group 8). ^{a} Approximate targeted cell count. ^{b} Approximate dose volume. ^{c} PBS injected subretinally in the right eye and intravitreally in the left eye ^{d} These animals were followed for approximately 4.5 months. | | | | | |

Docket No. CEN5174USNP

**Table 10: shows the summary of the antibody responses in Sd rat injected with various cells of BDIX origin.**

| Recipient/strain | Donor cell | Allo/Xeno | Injection | Activated | Imm Suppr | Anti UTC IgG |
|---|---|---|---|---|---|---|
| Rat/SD | rat UTC | allo | S.C. | no | no | No |
| Rat/SD | rat PBMC | allo | S.C. | no | no | Yes * |
| Rat/SD | rat splenocytes | allo | S.C. | no | no | Yes * |
| Rat/SD | human UTC | xeno | S.C. | no | no | Yes |

**Table 11: shows antibody responses in various combination of cells recipients and cells injected.**

| Recipient/strain | Donor cell | Allo/Xeno | Injection | Activated | Imm Suppr | anti UTC IgG |
|---|---|---|---|---|---|---|
| Rat/SD | -at UTC | allo | jugular | yes | yes/CSA | no |
| Rat/SD | rat UTC | allo | I.C + occl | no | no | yes |
| Rat/SD | rat BMDC | allo | I.C + occl | no | no | yes |
| Rat/SD | human UTC | xeno | S.C. | no | yes/CSA | no |
| Rat/RCS | human UTC | xeno | S.C. | no | yes/CSA | no |
| Rat/RCS | human UTC | xeno | S.C. | no | no | yes |
| Rat/RCS | human UTC | xeno | S.R. | no | yes/CSA | no |
| Rat/Nude | human UTC | xeno | jugular | no | no | no** |
| Rat/Nude | human UTC | xeno | S.C. | no | no | no** |
| Rat/SD | human MSC | xeno | S.C. | no | yes/CSA | no |
| Rat/SD | human MSC | xeno | S.C. | no | no | yes |

**Table 12, Summary of pigs enrolled in immunogenicity study for multiple s.c. injections.**

| Immunosuppression | Time interval (in months) | Number of injections | Location of injections | Pigs per group | Animal ID |
|---|---|---|---|---|---|
| none | 3 | 2 | Same site | 2 | 17164 |
| | | | | | 17165 |
| none | 6 | 2 | Same site | 2 | 17162 |
| | | | | | 17163 |
| prednisone at 2^{nd} injection | 1 | 2 | Same site | 2 | 17307 |
| | | | | | 17308 |
| CsA & prednisone at both injections | 1 | 2 | Same site | 2 | 17375 |
| | | | | | 17377 |
| none | 1 | 2 | Separate site | 2 | 17471 |
| | | | | | 17475 |

**Table 13, Summary of all immune assessment assays results for the pig studies: MLR, serum antibody detection, and T cell mediated cytotoxicity. *IgM present, possibly due to pre-existing infection.**

| Animal | Immunosuppression | Time Interval Location | D2 or D3 Response to DD PBMC 1^{st} inj. | D2 or D3 Response to DD PBMC | Antibody Response Post-1^{st} injection | Antibody Response Post- 2^{nd} Injection |
|---|---|---|---|---|---|---|
| 17164 | none | 3-month Same site | no | no | no | yes |
| 17165 | none | 3-month Same site | no | no | no | yes |
| 17162 | none | 6-month Same site | no | no | no | yes |
| 17163 | none | 6-month Same site | no | no | no | yes |
| 17307 | predisone 2^{nd} injection only | 1-month Same site | no | no | no | yes |
| 17308 | predisone 2^{nd} injection only | 1-month Same site | no | no | no | yes |
| 17375 | Predisone & CsA 1^{st} & 2^{nd} injections | 1-month Same site | no | no | no | no |
| 17377 | Predisone & CsA 1^{st} & 2^{nd} injections | 1-month Same site | no | no | no | no |
| 17471 | none | 1-month Separate site | no | no | *yes | yes |
| 17475 | none | 1-month Separate site | no | no | no | yes |

**Table 14, Summary of skin graft rejection tempos in relation to treatment group and the presence of serum antibodies prior to skin grafting. * After 2^{nd} injection.**

| **Animal** | **Immunosuppression** | **Time Interval (Months)** | **Antibody before skin graft *** | **Clinical Pattern of Skin Graft Rejection (start to completion day)** | **Antibody after skin graft** |
|---|---|---|---|---|---|
| 17164 | none | 3 | Yes - IgG | Accelerated | Yes - IgG |
| | | | | (1-7) | |
| 17165 | none | 3 | Yes - IgG | Accelerated | Yes - IgG |
| | | | | (1-7) | |
| 17162 | none | 6 | Yes - IgG | Accelerated | Yes - IgG |
| | | | | (1-7) | |
| 17163 | none | 6 | Yes - IgG | Accelerated | Yes - IgG |
| | | | | (1-7) | |
| 17307 | Predisone at 2^{nd} injection only | 1 | Yes - IgG | Accelerated | Yes - IgG |
| | | | | (1-7) | |
| 17308 | Predisone at 2^{nd} injection only | 1 | Yes - IgG | Accelerated | Yes - IgG |
| | | | | (1-7) | |
| 17375 | Predisone & CsA at both injections | 1 | No | Normal | Yes - IgG |
| | | | | (4-8) | |
| 17378 | Predisone & CsA at both injections | 1 | No | Normal | Yes - IgG |
| | | | | (4-8) | |
| 17471 | none | 1 | Yes - IgG | Accelerated | Yes - IgG |
| | | (separate site) | | (1-7) | |
| 17475 | none | 1 | Yes - IgG | Accelerated | Yes - IgG |
| | | (separate site) | | (1-7) | |

**Table 15, Summary of pigs enrolled in immunogenicity study for multiple s.r. injections.**

| Injected cells | Time interval (in months) | Number of injections | Pigs per group | Animal ID |
|---|---|---|---|---|
| pUTC | 6 | 2 | 3 | 351-17225 |
| | | | | 352-17161 |
| | | | | 352-17129 |
| pUTC | 3 | 3 | 3 | 201-17128 |
| | | | | 251-17227 |
| | | | | 252-17226 |
| pPBMC after SC priming with PBMC | 3 | 3 | 2 | 101-17135 |
| | | | | 151-17242 |

## Claims

1. A method to determine the optimal administration protocol of allogeneic donor tissue to treat a disease in a human, using an animal model of human disease, comprising the steps of:
a. Obtaining allogeneic donor tissue from a donor animal,
b. Inducing a disease state in a recipient animal,
c. Administering the allogeneic donor tissue to the recipient animal, and monitoring the immune response elicited by the allogeneic donor tissue, and
d. Altering the administration of the allogeneic donor tissue to reduce the immune response in the recipient animal.

2. The method of claim 1 further comprising the step of monitoring the efficacy of the allogeneic donor tissue in the recipient animal, inserted between steps c. and d.

3. The method of claim 1 or 2, wherein:
i) the disease state in the recipient animal is induced experimentally; or
ii) the disease state in the recipient animal is genetic.

4. The method of any preceding claim, wherein the immune response elicited by the allogeneic donor tissue is:
i) any immune response; and/or
ii) rejection of the allogeneic donor tissue; and/or
iii) an inflammatory response.

5. The method of claim 4iii), wherein the inflammatory response is mediated by interferon-γ.

6. The method of claim 5, wherein the inflammatory response that is mediated by interferon- y is an increase in the expression of:
i) MHC class II on the allogeneic donor tissue; or
ii) MHC class I and II on the allogeneic donor tissue.

7. The method of any preceding claim, wherein the administration is altered by altering the amount of allogeneic donor tissue administered to the recipient animal.

8. The method of claim 7, wherein:
i) the amount of allogeneic donor tissue per administration is altered; and/or
ii) the number of administrations of allogenic donor tissue is altered.

9. The method of any preceding claim, wherein the administration is altered by altering the timing of the administration of the allogeneic donor tissue administered to the recipient animal.

10. The method of claim 9, wherein:
i) the timing of the initial administration to the recipient animal is altered; and/or
ii) the timing of any subsequent administration to the recipient animal is altered.

11. The method of any preceding claim, wherein the administration is altered by altering the site of the administration of the allogeneic donor tissue administered to the recipient animal.

12. The method of any preceding claim, wherein the recipient animal is treated with at least one agent that reduces the immune response elicited by the allogeneic donor tissue.

13. The method of claim 12, wherein:
i) the recipient animal is treated systemically; and/or
ii) the recipient animal is treated at the site of administration; and/or
iii) the recipient animal is treated prior to administration of the allogeneic donor tissue; and/or
iv) the recipient animal is treated after the administration of the allogeneic donor tissue.

14. The method of any preceding claim, wherein the allogeneic donor tissue is treated with at least one agent that reduces the immune response elicited by the allogeneic donor tissue.

15. The method of any preceding claim, wherein the immune response observed in the recipient animal in response to the allogeneic donor tissue is predicted to be the same as the probable immune response observed in the recipient human in response to the allogeneic donor tissue.

16. The method of any preceding claim, wherein the allogeneic donor tissue obtained from a donor animal is equivalent to the allogeneic donor tissue obtained from a donor human.

17. The method of any preceding claim, wherein the allogeneic donor tissue comprises cells, for example umbilicus-derived cells.
